# EUROPEAN PATENT APPLICATION

(11) **EP 2 352 107 A1**
(43) Date of publication of application: **03.08.2011**
(21) Application number: 09820370.6
(22) Date of filing: 16.09.2009
(51) Int. Cl.: G06F 17/50

(54) **CHEMICAL REACTION TRANSITION STATE SEARCH SYSTEM, METHOD, AND PROGRAM**

(30) Priority: 15.10.2008 JP 2008266903
(71) Applicant: Yamaguchi University, Yamaguchi 753-8511 (JP)
(72) Inventor: HORI, Kenji, Ube-shi Yamaguchi 755-8611 (JP); YAMAGUCHI, Toru, Ube-shi Yamaguchi 755-8611 (JP)
(74) Representative: Plougmann & Vingtoft A/S
(86) International application number: PCT/JP2009/004656
(87) International publication number: WO 2010/044191

(57) **Abstract**

[OBJECT] A chemical reaction transition state search system, method, and program are provided in which a target TS can be completely obtained even if the molecular structure of a reaction product to be synthetically produced becomes complex.

[ACHIEVING MEANS] The invention is a chemical reaction transition state search system 1 that includes an input device 2, an arithmetic processing unit 3, and a storage device 5 in order to find a chemical structure being in a targeted transition state in a chemical reaction (hereinafter, a chemical structure being in a transition state is referred to as a transition-state geometry or a TS, and a transition-state geometry to be targeted is referred to as a target transition-state geometry or a target TS).

## Description

### Technical Field

This invention relates to a chemical reaction transition state search system for searching for a transition state occurring during a chemical reaction to synthetically produce a compound, relates to a method therefor, and relates to a program therefor.

### Background Art

In order to create new molecules having useful functions or useful reactivity, it is important to perform rational molecular design or rational reaction design before creating these molecules. In general, when the reaction design is performed, an optimal synthetic route is selected depending on the amount of benefit obtainable from a plurality of synthetic routes, and the greatest problem relative to this benefit is whether a reaction easily occurs or not.
Although the simplest, most effective method for judging whether a reaction easily occurs in a synthetic route is to search for a transition state included therein and to evaluate activation energy, an actual search for such a transition state becomes more difficult in proportion to the increasing complexity of a molecular structure, and has existed as a high hurdle that is faced when a novel compound, such as a medicine, is created.

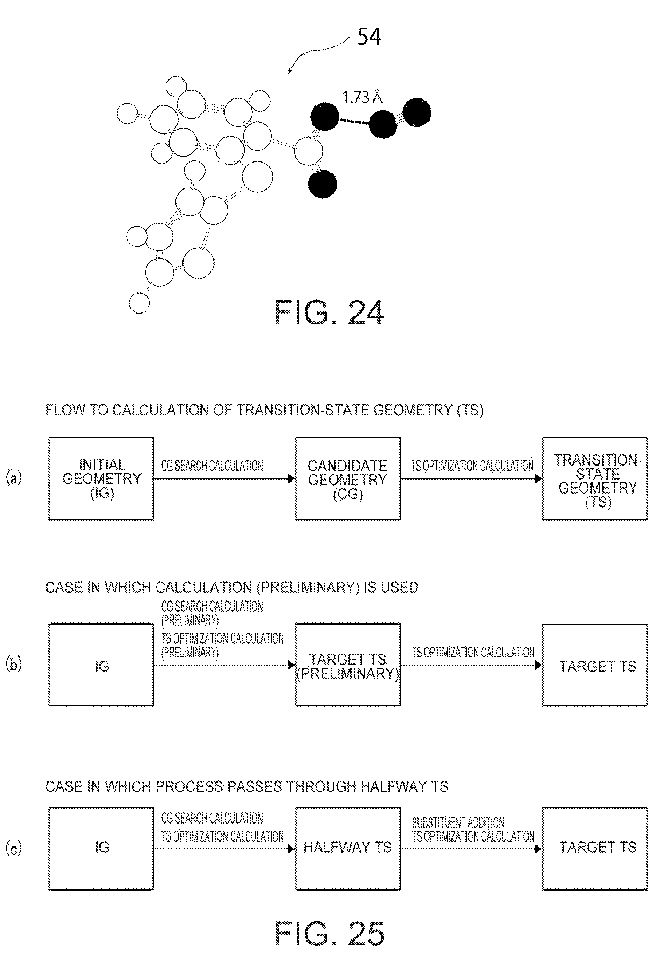

A conventional technique relative to a transition state search method will be described with reference to FIG. 25.
(a) of FIG. 25 is a flowchart showing a general flow from an initial geometry (IG) to the calculation of a transition-state geometry (TS) through a candidate geometry (CG) of the transition-state geometry, and (b) of FIG. 25 is a flowchart showing a flow in which a target TS (preliminary) is analyzed from an IG by performing a calculation (preliminary), and, in addition, the target TS is calculated through an optimization calculation, and (c) of FIG. 25 is a flowchart showing a flow in which a halfway TS, not the target TS, is temporarily analyzed from the IG, and then the target TS is analyzed and calculated. The calculation (preliminary), the target TS, and the halfway TS will be described later. In the present patent application, the term provided with " (preliminary) " following the "TS" denotes a TS obtained as a result of a calculation (preliminary), and is distinguished as a mere TS that is obtained by a "mere calculation" described later.
   In FIG. 25, (b) and (c) show a case in which a target TS (preliminary) and a halfway TS are employed as a CG of (a) . In FIG. 25, first, there is a need to form a structure that serves as a starting point where a calculation is performed to find a transition state in order to obtain a transition-state geometry (TS) to be targeted (see left side of (a) of FIG. 25). This is generally called an "initial geometry" (IG), and is often formed as an intermediate structure between the structure of a reactant in a reaction and the structure of a product.

A structure close to a transition state is inevitably required to find this transition state. Herein, this structure close to the transition state is called a "CG" as a candidate geometry of a transition-state geometry as shown in the center of (a) of FIG. 25. After forming an IG, a calculation is performed to search for this CG. This is called a "CG search calculation." Some calculation methods, such as a minimum energy path method, a saddle method, and a contour method, are employed to perform the CG search calculation. The minimum energy path method mentioned here is a calculation method in which a structure obtained by changing a structure near reactive sites in a step-by-step manner is generated based on an IG and in which a search for a CG is performed while calculating the energy of its structure.
After having found the CG, a TS optimization calculation is performed to find a transition-state geometry (TS) based on the structure of the CG (see right side of (a) of FIG. 25). A TS can be found by performing this optimization calculation. Mechanically, whether a TS has been found can be confirmed by performing a vibration analysis of the TS. If normal vibrations having imaginary frequency solely exist, this is a TS.
An analysis to find these transition states is performed by a quantum chemical calculation, and is performed chiefly according to three kinds of calculation methods, namely,
(1) semi-empirical molecular orbital calculation method,
(2) non-empirical molecular orbital calculation method, and
(3) density-functional-theory calculation method.

These calculation methods have two kinds of calculations, i.e., a mere calculation (in other words, a calculation having high accuracy) and a preliminary calculation (hereinafter, referred to as a "calculation (preliminary)"). The "calculation (preliminary)" denotes a calculation lower in accuracy than the mere calculation, and, in general, has a shorter calculation time than the mere calculation. Being low in accuracy denotes that the error between a calculation result and an actual measured value is great, whereas being high in accuracy denotes that the error therebetween is small.
In the above-mentioned calculation methods (1) to (3), the semi-empirical molecular orbital calculation (e.g., AM1, PM3, etc.) or the non-empirical molecular orbital calculation that has a low level (e.g., HF/STO-3G, HF/3-21G, etc.) is used as a calculation (preliminary) low in accuracy, whereas the density-functional-theory calculation (e.g., B3LYP/6-31G*, B3PW91/cc-cpVQZ) or the non-empirical molecular orbital calculation that has a high level (e.g., MP2/6-311+G**, CASSCF/aug-cc-pVQZ) is used as a calculation high in accuracy. In the present patent application, the non-empirical molecular orbital calculation that has a high level is a highly accurate calculation whose calculation level is HF/6-31G or greater, whereas the empirical molecular orbital calculation that has a low level is a low-accuracy calculation whose calculation level does not exceed HF/3-21G.

As shown in (b) of FIG. 25, in order to shorten the time taken when a TS (a target TS) to be found is calculated, a calculation (preliminary) is first performed, i.e., a CG search calculation (preliminary) and a TS optimization calculation (preliminary) are first performed, and a target TS (preliminary) is found by the calculation (preliminary) . The target TS (preliminary) is low in accuracy, and therefore a TS optimization calculation is performed to increase its accuracy so as to find a target TS.
Generally, it becomes more difficult to find a target TS in proportion to the increasing complexity of its molecular structure, and the target TS cannot be found by a usual method in many cases. As a conventional method that copes with such cases, there is a method for finding a target TS via a halfway TS as shown in (c) of FIG. 25. This method makes it possible to obtain a target TS by performing a CG search calculation and a TS optimization calculation, by proceeding via a halfway TS, and by adding a substituent thereto.

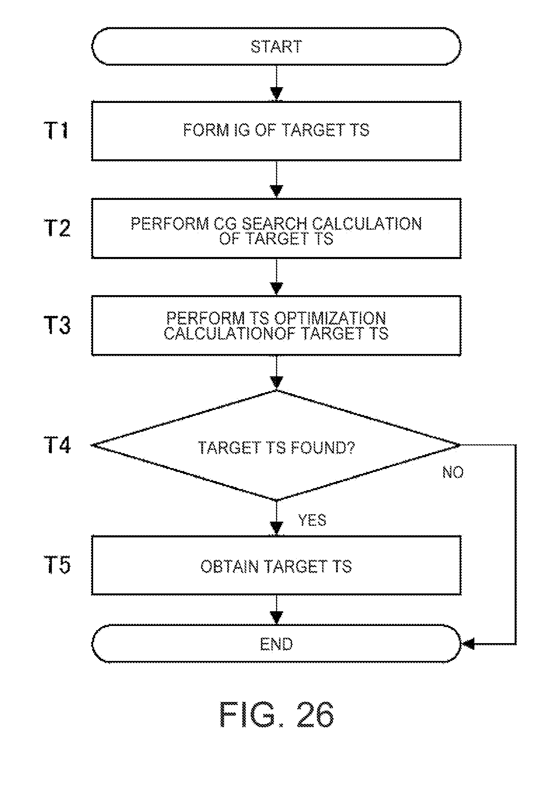

Next, a method for analyzing a transition-state geometry will be described in detail with reference to FIG. 26 to FIG. 28.
FIG. 26 is a flowchart showing a conventional technique of a transition state search method that is the most basic one.
In FIG. 26, an IG of a target TS is formed at step T1, and a CG search calculation to find a CG is performed by use of the IG at step T2. In this CG search calculation, a highly accurate quantum chemical calculation is performed. Thereafter, at step T3, a TS optimization calculation is performed to find the target TS by use of the CG obtained at step T2. At step T4, it is determined whether the target TS has been found by the calculation performed at step T3, and, if the target TS has been found, the target TS is obtained at step T5, and, if the target TS has not been found, the analysis is ended.

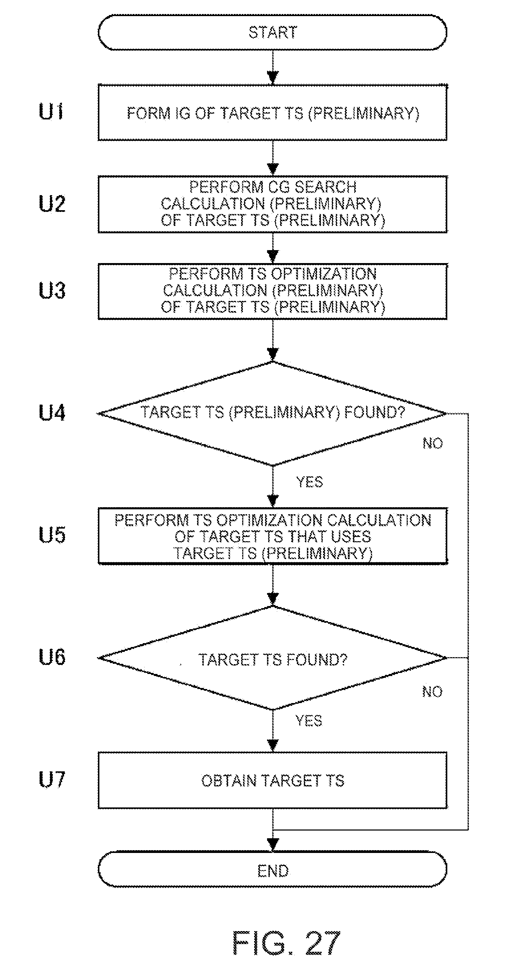

FIG. 27 is a flowchart which shows a conventional technique of a transition state search method that uses a calculation (preliminary) in order to make a calculation time shorter than in the conventional technique of FIG. 26, and in which the method of (b) of FIG. 25 is shown in a condensed flow form.
In FIG. 27, to use a calculation (preliminary), an IG to search for a target TS (preliminary) is formed at step U1. Thereafter, a CG search calculation (preliminary) to find the target TS (preliminary) is performed at step U2, and, after CG has been searched, a TS optimization calculation (preliminary) is performed at step U3, and it is determined whether the target TS (preliminary) has been found, and, as a result, the target TS (preliminary) is obtained at step U4. If the target TS (preliminary) is not obtained at step U4, the analysis is ended, and a target TS cannot also be obtained. If the target TS (preliminary) is obtained at step U4, a TS optimization calculation is performed based on its structure at step U5, and it is determined at step U6 whether the target TS has been found, and the target TS is obtained at step U7. However, there is a case in which the target TS is not obtained at step U6, and, if so, the analysis is ended.
In the analysis shown in FIG. 27, the calculation (preliminary) is employed to first find the target TS (preliminary), and therefore, advantageously, a calculation time is shortened. However, on the other hand, disadvantageously, there has been a case in which it is often impossible to obtain a target TS of a reaction product that has a complex molecular structure.

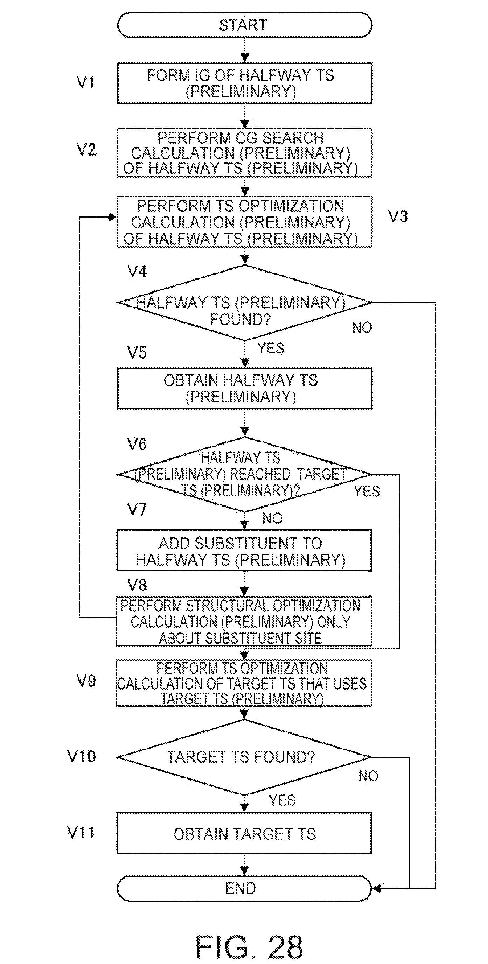

Therefore, a conventional technique shown in FIG. 28 is a method developed to obtain such a target TS of a reaction product that has a complex molecular structure. This conventional technique employs a procedure in which a halfway TS (preliminary) is found by use of a calculation (preliminary), and then a target TS (preliminary)is found via this halfway TS (preliminary), and furthermore a target TS is found. The halfway TS has a molecular structure in which the structure of the target TS is simplified while retaining a skeletal structure of the target TS (which is a basic part of the structure of the target TS and which is a structure simplified by removing a substituent).
In FIG. 28, in order to find a halfway TS, an IG required to find a halfway TS (preliminary) is first formed at step V1. Thereafter, a CG search calculation (preliminary) and a TS optimization calculation (preliminary) are performed (steps V2 and V3), and it is determined at step V4 whether the halfway TS (preliminary) has been found, and the halfway TS (preliminary) is obtained (step V5).
Thereafter, it is confirmed whether the halfway TS (preliminary) is the same in structure as the target TS (preliminary) (step V6), and, if a structural shortage exists, a suitable substituent is added to the structure (step V7), and a structural optimization calculation (preliminary) of only the site of the substituent added thereto is performed (step V8).
Thereafter, the process returns to step V3, and a TS optimization calculation (preliminary) is performed in the same way as before. When the structure of the halfway TS (preliminary) and that of the target TS (preliminary) coincide with each other, a TS optimization calculation that is not preliminary is performed (step V9), and then it is determined whether the target TS has been found (step V10), and the target TS is obtained (step V11). If the target TS is not found at step V10, the analysis is ended.
In this conventional technique, it has become possible to find the target TS by undergoing the halfway TS (preliminary) even if its molecular structure is complex to some degree. However, in proportion to the increasing complexity of the molecular structure, it has become more difficult to find the target TS at the TS optimization calculation part of this target TS, and there has remained the possibility of the frequent occurrence of a case in which the target TS cannot be found.
This conventional technique is also introduced in Non-Patent Documents 1 and 2.

### Prior Art Documents

### Non-Patent Documents

Non-Patent Document 1: Hori Kenji, Yamazaki Suzuko, "Computational Chemical Experiment", Maruzen (1998) pp.33-49, pp.115-117
Non-Patent Document 2: Hori Kenji, Yamazaki Suzuko, "Information Chemistry·Computational Chemical Experiment," Maruzen (2006) pp.93-105

### BRIEF SUMMARY OF THE INVENTION

### Problems to be Solved by the Invention

It is therefore an object of the present invention to provide a chemical reaction transition state search system, a chemical reaction transition state search method, and a chemical reaction transition state search program according to each of which a target TS can be substantially completely obtained even if the molecular structure of a reaction product to be synthetically produced becomes complex.

### Means for Solving the Problems

To achieve the object, a chemical reaction transition state search system that is an invention according to claim 1 is a chemical reaction transition state search system including an input device, an arithmetic processing unit, and a storage device in order to find a chemical structure being in a targeted transition state in a chemical reaction (hereinafter, a chemical structure being in a transition state is referred to as a transition-state geometry or a TS, and a transition-state geometry to be targeted is referred to as a target transition-state geometry or a target TS), characterized in that the arithmetic processing unit includes an IG forming unit that receives an input of an original structure of a halfway transition-state geometry being in a stage prior to the target TS (hereinafter, a "halfway transition-state geometry" is referred to specifically as a "halfway TS") and then calculates an initial geometry (hereinafter, referred to as an "IG") serving as a starting point to find the halfway TS from the halfway-TS original structure; a CG-search calculating unit that calculates a candidate geometry (hereinafter, referred to as a "CG" ) close to a transition state from an IG formed by the IG forming unit; a TS optimization calculating unit that determines whether an obtained TS is a true TS or is an original structure of a TS in such a manner that the TS is found by calculating a molecular structure that maximizes all energy while changing the molecular structure with respect to a CG calculated by the CG-search calculating unit or with respect to the original structure of the TS calculated from the resulting CG, and the resulting TS is subjected to a vibration analysis by use of a vibration analytic function model; a reactive-site fixing unit that fixes a reactive site of the original structure of the TS; a substituent processing unit that adds or removes a substituent to or from the original structure of the TS; and a structural optimization calculating unit that calculates optimization of a molecular structure forming the original structure of the TS by calculating the molecular structure that minimizes all energy while changing the molecular structure; and characterized in that the storage device stores structure data of the IG, of the CG, and of the TS, reactive-site data, and substituent data, and the CG-search calculating unit, the TS optimization calculating unit, and the structural optimization calculating unit selectively include both a functional model used for calculations and a functional model used to perform a calculation lower in accuracy than the functional model used for calculations.

A chemical reaction transition state search system that is an invention according to claim 2 is a chemical reaction transition state search system including an input device, an arithmetic processing unit, and a storage device in order to find a chemical structure being in a targeted transition state in a chemical reaction (hereinafter, a chemical structure being in a transition state is referred to as a transition-state geometry or a TS, and a transition-state geometry to be targeted is referred to as a target transition-state geometry or a target TS), characterized in that the arithmetic processing unit includes a halfway-TS setting unit that calculates an original structure of a halfway transition-state geometry being in a stage prior to the target TS (hereinafter, a "halfway transition-state geometry" is referred to specifically as a "halfway TS") by receiving an input of data relative to a product of the chemical reaction; an IG forming unit that calculates an initial geometry (hereinafter, referred to as an "IG") serving as a starting point to find the halfway TS from the halfway-TS original structure set by the halfway-TS setting unit; a CG-search calculating unit that calculates a candidate geometry (hereinafter, referred to as a CG ) close to a transition state from an IG formed by the IG forming unit; a TS optimization calculating unit that determines whether an obtained TS is a true TS or is an original structure of a TS in such a manner that the TS is found by calculating a molecular structure that maximizes all energy while changing the molecular structure with respect to a CG calculated by the CG-search calculating unit or with respect to the original structure of the TS calculated from the resulting CG, and the resulting TS is subjected to a vibration analysis by use of a vibration analytic function model; a reactive-site fixing unit that fixes a reactive site of the original structure of the TS; a substituent processing unit that adds or removes a substituent to or from the original structure of the TS; and a structural optimization calculating unit that calculates optimization of a molecular structure forming the original structure of the TS by calculating the molecular structure that minimizes all energy while changing the molecular structure; and characterized in that the storage device stores structure data of the IG, of the CG, and of the TS, reactive-site data, and substituent data, and the CG-search calculating unit, the TS optimization calculating unit, and the structural optimization calculating unit selectively include both a functional model used for calculations and a functional model used to perform a calculation lower in accuracy than the functional model used for calculations.

The invention according to claim 3 is characterized in that the chemical reaction transition state search system according to claim 1 or claim 2 further includes an output device that outputs or displays structure data relative to the TS that has been regarded as a true TS in the TS optimization calculating unit to or on an external device.

The invention according to claim 4 is a chemical reaction transition state search method for finding a chemical structure being in a targeted transition state in a chemical reaction (hereinafter, a chemical structure being in a transition state is referred to as a transition-state geometry or a TS, and a transition-state geometry to be targeted is referred to as a target transition-state geometry or a target TS), the chemical reaction transition state search method characterized by obtaining a halfway TS (hereinafter, a halfway TS obtained by a calculation (preliminary) is referred to as a "halfway TS (preliminary)") from an original structure of a halfway transition-state geometry being in a stage prior to the target TS (hereinafter, a "halfway transition-state geometry" is referred to specifically as a "halfway TS") by calculating a molecular structure that maximizes all energy while changing the molecular structure according to a calculation (preliminary) using a functional model lower in accuracy; and obtaining the target TS by adding a substituent to the halfway TS (preliminary) so as to be a target TS (preliminary) and then by calculating the molecular structure that maximizes all energy while changing the molecular structure according to a calculation using a functional model higher in accuracy than the calculation (preliminary); and characterized by, if the target TS is not obtained, obtaining the halfway TS from the halfway TS (preliminary) by calculating the molecular structure that maximizes all energy while changing the molecular structure according to a calculation using the functional model higher in accuracy than the calculation (preliminary); and obtaining the target TS by adding a substituent to the halfway TS and by calculating the molecular structure that maximizes all energy while changing the molecular structure according to a calculation using the functional model higher in accuracy than the calculation (preliminary).

The invention according to claim 5 is a chemical reaction transition state search method for finding a chemical structure being in a targeted transition state in a chemical reaction (hereinafter, a chemical structure being in a transition state is referred to as a transition-state geometry or a TS, and a transition-state geometry to be targeted is referred to as a target transition-state geometry or a target TS), the chemical reaction transition state search method characterized by obtaining a halfway TS (hereinafter, a halfway TS obtained by a calculation (preliminary) is referred to as a "halfway TS (preliminary)") by calculating an original structure of a halfway transition-state geometry being in a stage prior to the target TS (hereinafter, a "halfway transition-state geometry" is referred to specifically as a "halfway TS") and by calculating a molecular structure that maximizes all energy while changing the molecular structure from the original structure of the halfway TS according to a calculation (preliminary) using a functional model lower in accuracy; and obtaining the target TS by adding a substituent to the halfway TS (preliminary) so as to be a target TS (preliminary) and then by calculating the molecular structure that maximizes all energy while changing the molecular structure according to a calculation using a functional model higher in accuracy than the calculation (preliminary); and characterized by, if the target TS is not obtained, obtaining the halfway TS from the halfway TS (preliminary) by calculating the molecular structure that maximizes all energy while changing the molecular structure according to a calculation using the functional model higher in accuracy than the calculation (preliminary); and obtaining the target TS by adding a substituent to the halfway TS and by calculating the molecular structure that maximizes all energy while changing the molecular structure according to a calculation using the functional model higher in accuracy than the calculation (preliminary).

The invention according to claim 6 is a chemical reaction transition state search method for finding a chemical structure being in a targeted transition state in a chemical reaction (hereinafter, a chemical structure being in a transition state is referred to as a transition-state geometry or a TS, and a transition-state geometry to be targeted is referred to as a target transition-state geometry or a target TS), the chemical reaction transition state search method comprising an IG forming step (S1) of receiving an input of a halfway-TS original structure of a halfway transition-state geometry being in a stage prior to the target TS (hereinafter, a "halfway transition-state geometry" is referred to specifically as a "halfway TS") and then calculating an initial geometry (hereinafter, referred to as an "IG") serving as a starting point to find the halfway TS from the halfway-TS original structure; a CG-search calculating step (S2) of calculating a candidate geometry (hereinafter, referred to as a "CG") close to a transition state from an IG formed by the IG forming step according to a calculation (preliminary) using a functional model lower in accuracy (hereinafter, a CG obtained by the calculation (preliminary) is referred to as a "CG (preliminary)"); a halfway TS (preliminary) optimization calculating step (S3) of obtaining a halfway TS (hereinafter, a halfway TS obtained according to the calculation (preliminary) is referred to as a "halfway TS (preliminary) ") by calculating a molecular structure that maximizes all energy while changing the molecular structure with respect to the CG (preliminary) obtained by the CG-search calculating step according to the calculation (preliminary) using a functional model lower in accuracy; a halfway TS (preliminary) determining step (S4) of determining whether an obtained halfway TS (preliminary) is a true halfway TS (preliminary) or is an original structure of a halfway TS (preliminary) in such a manner that the obtained halfway TS (preliminary) is subjected to a vibration analysis by use of a vibration analytic function model; a writing step (S5) of readably writing a halfway TS (preliminary) obtained when it is determined that the obtained halfway TS (preliminary) is a halfway TS (preliminary) at the halfway TS (preliminary) determining step onto the storage device; a target TS (preliminary) determining step (S6) of determining whether the halfway TS (preliminary) coincides with an original structure of a target TS (preliminary); a target TS (preliminary) structural optimization calculating step (S9) of, when the halfway TS (preliminary) coincides with the original structure of the target TS (preliminary), calculating optimization of a molecular structure forming the target TS (preliminary) by fixing a reactive site of the target TS (preliminary) and by calculating the molecular structure that minimizes all energy while changing the molecular structure forming the target TS (preliminary) according to the calculation (preliminary) using a functional model lower in accuracy; a target TS optimization calculating step (S10) of obtaining a target TS by unfixing the fixed reactive site of the target TS (preliminary) whose molecular structure has been optimized and by calculating a molecular structure that maximizes all energy while changing the molecular structure according to a calculation using a functional model higher in accuracy than the calculation (preliminary) ; a first substituent adding step (S7) of adding a substituent to the halfway TS (preliminary) when the halfway TS (preliminary) does not coincide with the original structure of the target TS (preliminary) at the target TS (preliminary) determining step (S6) ; a first substituent structural optimization calculating step (S8) of calculating optimization of a molecular structure of a substituent by calculating a molecular structure that minimizes all energy while changing the molecular structure with respect to only a site of the added substituent according to a calculation (preliminary) using a functional model lower in accuracy and then incorporating the resulting structure into the halfway TS (preliminary) optimization calculating step (S3) as a new halfway TS (preliminary); a first target TS determining step (S11) of determining whether the target TS obtained at the target TS optimization calculating step (S10) is a true target TS or is an original structure of the target TS by performing a vibration analysis of the obtained target TS by use of a vibration analytic function model; a halfway TS structural optimization calculating step (S14) of, when it is determined that the obtained target TS is the original structure of the target TS at the first target TS determining step (S11), calculating optimization of a molecular structure forming the halfway TS (preliminary) by performing a reading step (S13) of reading the halfway TS (preliminary) written onto the storage device at the writing step (S5), by fixing a reactive site of the halfway TS (preliminary), and by calculating the molecular structure that minimizes all energy while changing the molecular structure according to a calculation using a functional model higher in accuracy than the calculation (preliminary); a first halfway TS optimization calculating step (S15) of obtaining a halfway TS by unfixing the fixed reactive site of the halfway TS whose molecular structure has been optimized and by calculating the molecular structure that maximizes all energy while changing the molecular structure according to a calculation using a functional model higher in accuracy than the calculation (preliminary); a first halfway TS determining step (S16) of determining whether an obtained halfway TS is a true halfway TS or is an original structure of the halfway TS by performing a vibration analysis of the obtained halfway TS by use of a vibration analytic function model and, when it is determined that the obtained halfway TS is the original structure of the halfway TS, incorporating a halfway TS (preliminary) written prior to the halfway TS (preliminary) written onto the storage device into the reading step (S13); a second target TS determining step (S18) of, when it is determined (S17) that the obtained halfway TS is a true halfway TS at the first halfway TS determining step (S16), determining whether the halfway TS coincides with the target TS; a second substituent adding step (S19) of, when it is determined that the halfway TS does not coincide with the target TS at the second target TS determining step (S18), adding a substituent to the halfway TS; a second substituent structural optimization calculating step (S20) of calculating optimization of a molecular structure of a substituent by calculating a molecular structure that minimizes all energy while changing the molecular structure with respect to only a site of the added substituent according to a calculation using a functional model higher in accuracy than the calculation (preliminary); a second halfway TS optimization calculating step (S21) of obtaining a halfway TS by calculating a molecular structure that maximizes all energy while changing the molecular structure according to a calculation using a functional model higher in accuracy than the calculation (preliminary) while setting the halfway TS optimized at the second substituent structural optimization calculating step as a new halfway TS; and a second halfway TS determining step (S22) of determining whether the halfway TS obtained at the second halfway TS optimization calculating step (S21) is a true halfway TS or is an original structure of the halfway TS by performing a vibration analysis of the obtained halfway TS by use of a vibration analytic function model and, when it is determined that the obtained halfway TS is a true halfway TS, incorporating the halfway TS into the second target TS determining step (S18).

The invention according to claim 7 is a chemical reaction transition state search method for finding a chemical structure being in a targeted transition state in a chemical reaction (hereinafter, a chemical structure being in a transition state is referred to as a transition-state geometry or a TS, and a transition-state geometry to be targeted is referred to as a target transition-state geometry or a target TS), the chemical reaction transition state search method comprising a halfway TS setting step (S0) of calculating an original structure of a halfway transition-state geometry being in a stage prior to the target TS (hereinafter, a "halfway transition-state geometry" is referred to specifically as a "halfway TS"); an IG forming step (S1) of calculating an initial geometry (hereinafter, referred to as an "IG") that serves as a starting point to find the halfway TS from the halfway-TS original structure set at the halfway TS setting step (S0); a CG-search calculating step (S2) of calculating a candidate geometry (hereinafter, referred to as a "CG") close to a transition state from an IG formed by the IG forming step according to a calculation (preliminary) using a functional model lower in accuracy (hereinafter, a CG obtained by the calculation (preliminary) is referred to as a "CG (preliminary)"); a halfway TS (preliminary) optimization calculating step (S3) of obtaining a halfway TS (hereinafter, a halfway TS obtained according to the calculation (preliminary) is referred to as a "halfway TS (preliminary) ") by calculating a molecular structure that maximizes all energy while changing the molecular structure with respect to the CG (preliminary) obtained by the CG-search calculating step (S2) according to the calculation (preliminary) using a functional model lower in accuracy; a halfway TS (preliminary) determining step (S4) of determining whether an obtained halfway TS (preliminary) is a true halfway TS (preliminary) or is an original structure of a halfway TS (preliminary) in such a manner that the obtained halfway TS (preliminary) is subjected to a vibration analysis by use of a vibration analytic function model; a writing step (S5) of readably writing a halfway TS (preliminary) obtained when it is determined that the obtained halfway TS (preliminary) is a halfway TS (preliminary) at the halfway TS (preliminary) determining step onto the storage device; a target TS (preliminary) determining step (S6) of determining whether the halfway TS (preliminary) coincides with an original structure of a target TS (preliminary); a target TS (preliminary) structural optimization calculating step (S9) of, when the halfway TS (preliminary) coincides with the original structure of the target TS (preliminary), calculating optimization of a molecular structure forming the target TS (preliminary) by fixing a reactive site of the target TS (preliminary) and by calculating the molecular structure that minimizes all energy while changing the molecular structure forming the target TS (preliminary) according to the calculation (preliminary) using a functional model lower in accuracy; a target TS optimization calculating step (S10) of obtaining a target TS by unfixing the fixed reactive site of the target TS (preliminary) whose molecular structure has been optimized and by calculating a molecular structure that maximizes all energy while changing the molecular structure according to a calculation using a functional model higher in accuracy than the calculation (preliminary) ; a first substituent adding step (S7) of adding a substituent to the halfway TS (preliminary) when the halfway TS (preliminary) does not coincide with the original structure of the target TS (preliminary) at the target TS (preliminary) determining step (S6); a first substituent structural optimization calculating step (S8) of calculating optimization of a molecular structure of a substituent by calculating a molecular structure that minimizes all energy while changing the molecular structure with respect to only a site of the added substituent according to a calculation (preliminary) using a functional model lower in accuracy and then incorporating the resulting structure into the halfway TS (preliminary) optimization calculating step (S3) as a new halfway TS (preliminary); a first target TS determining step (S11) of determining whether the target TS obtained at the target TS optimization calculating step (S10) is a true target TS or is an original structure of the target TS by performing a vibration analysis of the obtained target TS by use of a vibration analytic function model; a halfway TS structural optimization calculating step (S14) of, when it is determined that the obtained target TS is the original structure of the target TS at the first target TS determining step (S11), calculating optimization of a molecular structure forming the halfway TS (preliminary) by performing a reading step (S13) of reading the halfway TS (preliminary) written onto the storage device at the writing step (S5), by fixing a reactive site of the halfway TS (preliminary), and by calculating the molecular structure that minimizes all energy while changing the molecular structure according to a calculation using a functional model higher in accuracy than the calculation (preliminary); a first halfway TS optimization calculating step (S15) of obtaining a halfway TS by unfixing the fixed reactive site of the halfway TS whose molecular structure has been optimized and by calculating the molecular structure that maximizes all energy while changing the molecular structure according to a calculation using a functional model higher in accuracy than the calculation (preliminary); a first halfway TS determining step (S16) of determining whether an obtained halfway TS is a true halfway TS or is an original structure of the halfway TS by performing a vibration analysis of the obtained halfway TS by use of a vibration analytic function model and, when it is determined that the obtained halfway TS is the original structure of the halfway TS, incorporating a halfway TS (preliminary) written prior to the halfway TS (preliminary) written onto the storage device into the reading step (S13); a second target TS determining step (S18) of, when it is determined (S17) that the obtained halfway TS is a true halfway TS at the first halfway TS determining step (S16), determining whether the halfway TS coincides with the target TS; a second substituent adding step (S19) of, when it is determined that the halfway TS does not coincide with the target TS at the second target TS determining step (S18), adding a substituent to the halfway TS; a second substituent structural optimization calculating step (S20) of calculating optimization of a molecular structure of a substituent by calculating a molecular structure that minimizes all energy while changing the molecular structure with respect to only a site of the added substituent according to a calculation using a functional model higher in accuracy than the calculation (preliminary); a second halfway TS optimization calculating step (S21) of obtaining a halfway TS by calculating a molecular structure that maximizes all energy while changing the molecular structure according to a calculation using a functional model higher in accuracy than the calculation (preliminary) while setting the halfway TS optimized at the second substituent structural optimization calculating step as a new halfway TS; and a second halfway TS determining step (S22) of determining whether the halfway TS obtained at the second halfway TS optimization calculating step (S21) is a true halfway TS or is an original structure of the halfway TS by performing a vibration analysis of the obtained halfway TS by use of a vibration analytic function model and, when it is determined that the obtained halfway TS is a true halfway TS, incorporating the halfway TS into the second target TS determining step (S18).

The invention according to claim 8 is a chemical reaction transition state search program executed by a computer in order to find a chemical structure being in a targeted transition state in a chemical reaction (hereinafter, a chemical structure being in a transition state is referred to as a transition-state geometry or a TS, and a transition-state geometry to be targeted is referred to as a target transition-state geometry or a target TS), the chemical reaction transition state search program allowing the computer to perform an IG forming step (S1) of receiving an input of an original structure of a halfway transition-state geometry being in a stage prior to the target TS (hereinafter, a "halfway transition-state geometry" is referred to specifically as a "halfway TS") and then calculating an initial geometry (hereinafter, referred to as an "IG") that serves as a starting point to find the halfway TS from the halfway-TS original structure; a CG-search calculating step (S2) of calculating a candidate geometry (hereinafter, referred to as a "CG") close to a transition state from an IG formed by the IG forming step according to a calculation (preliminary) using a functional model lower in accuracy (hereinafter, a CG obtained by the calculation (preliminary) is referred to as a "CG (preliminary)"); a halfway TS (preliminary) optimization calculating step (S3) of obtaining a halfway TS (hereinafter, a halfway TS obtained according to the calculation (preliminary) is referred to as a "halfway TS (preliminary)") by calculating a molecular structure that maximizes all energy while changing the molecular structure with respect to the CG (preliminary) obtained by the CG-search calculating step (S2) according to the calculation (preliminary) using a functional model lower in accuracy; a halfway TS (preliminary) determining step (S4) of determining whether an obtained halfway TS (preliminary) is a true halfway TS (preliminary) or is an original structure of a halfway TS (preliminary) in such a manner that the obtained halfway TS (preliminary) is subjected to a vibration analysis by use of a vibration analytic function model; a writing step (S5) of readably writing a halfway TS (preliminary) obtained when it is determined that the obtained halfway TS (preliminary) is a halfway TS (preliminary) at the halfway TS (preliminary) determining step onto the storage device; a target TS (preliminary) determining step (S6) of determining whether the halfway TS (preliminary) coincides with an original structure of a target TS (preliminary); a target TS (preliminary) structural optimization calculating step (S9) of, when the halfway TS (preliminary) coincides with the original structure of the target TS (preliminary), calculating optimization of a molecular structure forming the target TS (preliminary) by fixing a reactive site of the target TS (preliminary) and by calculating the molecular structure that minimizes all energy while changing the molecular structure forming the target TS (preliminary) according to the calculation (preliminary) using a functional model lower in accuracy; a target TS optimization calculating step (S10) of obtaining a target TS by unfixing the fixed reactive site of the target TS (preliminary) whose molecular structure has been optimized and by calculating a molecular structure that maximizes all energy while changing the molecular structure according to a calculation using a functional model higher in accuracy than the calculation (preliminary) ; a first substituent adding step (S7) of adding a substituent to the halfway TS (preliminary) when the halfway TS (preliminary) does not coincide with the original structure of the target TS (preliminary) at the target TS (preliminary) determining step (S6) ; a first substituent structural optimization calculating step (S8) of calculating optimization of a molecular structure of a substituent by calculating a molecular structure that minimizes all energy while changing the molecular structure with respect to only a site of the added substituent according to a calculation (preliminary) using a functional model lower in accuracy and then incorporating the resulting structure into the halfway TS (preliminary) optimization calculating step (S3) as a new halfway TS (preliminary); a first target TS determining step (S11) of determining whether the target TS obtained at the target TS optimization calculating step (S10) is a true target TS or is an original structure of the target TS by performing a vibration analysis of the obtained target TS by use of a vibration analytic function model; a halfway TS structural optimization calculating step (S14) of, when it is determined that the obtained target TS is the original structure of the target TS at the first target TS determining step (S11), calculating optimization of a molecular structure forming the halfway TS (preliminary) by performing a reading step (S13) of reading the halfway TS (preliminary) written onto the storage device at the writing step (S5), by fixing a reactive site of the halfway TS (preliminary), and by calculating the molecular structure that minimizes all energy while changing the molecular structure according to a calculation using a functional model higher in accuracy than the calculation (preliminary); a first halfway TS optimization calculating step (S15) of obtaining a halfway TS by unfixing the fixed reactive site of the halfway TS whose molecular structure has been optimized and by calculating the molecular structure that maximizes all energy while changing the molecular structure according to a calculation using a functional model higher in accuracy than the calculation (preliminary); a first halfway TS determining step (S16) of determining whether an obtained halfway TS is a true halfway TS or is an original structure of the halfway TS by performing a vibration analysis of the obtained halfway TS by use of a vibration analytic function model and, when it is determined that the obtained halfway TS is the original structure of the halfway TS, incorporating a halfway TS (preliminary) written prior to the halfway TS (preliminary) written onto the storage device into the reading step (S13); a second target TS determining step (S18) of, when it is determined (S17) that the obtained halfway TS is a true halfway TS at the first halfway TS determining step (S16), determining whether the halfway TS coincides with the target TS; a second substituent adding step (S19) of, when it is determined that the halfway TS does not coincide with the target TS at the second target TS determining step (S18), adding a substituent to the halfway TS; a second substituent structural optimization calculating step (S20) of calculating optimization of a molecular structure of a substituent by calculating a molecular structure that minimizes all energy while changing the molecular structure with respect to only a site of the added substituent according to a calculation using a functional model higher in accuracy than the calculation (preliminary); a second halfway TS optimization calculating step (S21) of obtaining a halfway TS by calculating a molecular structure that maximizes all energy while changing the molecular structure according to a calculation using a functional model higher in accuracy than the calculation (preliminary) while setting the halfway TS optimized at the second substituent structural optimization calculating step as a new halfway TS; and a second halfway TS determining step (S22) of determining whether the halfway TS obtained at the second halfway TS optimization calculating step (S21) is a true halfway TS or is an original structure of the halfway TS by performing a vibration analysis of the obtained halfway TS by use of a vibration analytic function model and, when it is determined that the obtained halfway TS is a true halfway TS, incorporating the halfway TS into the second target TS determining step (S18).

The invention according to claim 9 is a chemical reaction transition state search program executed by a computer in order to find a chemical structure being in a targeted transition state in a chemical reaction (hereinafter, a chemical structure being in a transition state is referred to as a transition-state geometry or a TS, and a transition-state geometry to be targeted is referred to as a target transition-state geometry or a target TS), the chemical reaction transition state search program allowing the computer to perform a halfway TS setting step (S0) of calculating an original structure of a halfway transition-state geometry being in a stage prior to the target TS (hereinafter, a "halfway transition-state geometry" is referred to specifically as a "halfway TS"); an IG forming step (S1) of calculating an initial geometry (hereinafter, referred to as an "IG") that serves as a starting point to find the halfway TS from the halfway-TS original structure set at the halfway TS setting step (S0); a CG-search calculating step (S2) of calculating a candidate geometry (hereinafter, referred to as a "CG") close to a transition state from an IG formed by the IG forming step according to a calculation (preliminary) using a functional model lower in accuracy (hereinafter, a CG obtained by the calculation (preliminary) is referred to as a "CG (preliminary)"); a halfway TS (preliminary) optimization calculating step (S3) of obtaining a halfway TS (hereinafter, a halfway TS obtained according to the calculation (preliminary) is referred to as a "halfway TS (preliminary)") by calculating a molecular structure that maximizes all energy while changing the molecular structure with respect to the CG (preliminary) obtained by the CG-search calculating step according to the calculation (preliminary) using a functional model lower in accuracy; a halfway TS (preliminary) determining step (S4) of determining whether an obtained halfway TS (preliminary) is a true halfway TS (preliminary) or is an original structure of a halfway TS (preliminary) in such a manner that the obtained halfway TS (preliminary) is subjected to a vibration analysis by use of a vibration analytic function model; a writing step (S5) of readably writing a halfway TS (preliminary) obtained when it is determined that the obtained halfway TS (preliminary) is a halfway TS (preliminary) at the halfway TS (preliminary) determining step onto the storage device; a target TS (preliminary) determining step (S6) of determining whether the halfway TS (preliminary) coincides with an original structure of a target TS (preliminary); a target TS (preliminary) structural optimization calculating step (S9) of, when the halfway TS (preliminary) coincides with the original structure of the target TS (preliminary), calculating optimization of a molecular structure forming the target TS (preliminary) by fixing a reactive site of the target TS (preliminary) and by calculating the molecular structure that minimizes all energy while changing the molecular structure forming the target TS (preliminary) according to the calculation (preliminary) using a functional model lower in accuracy; a target TS optimization calculating step (S10) of obtaining a target TS by unfixing the fixed reactive site of the target TS (preliminary) whose molecular structure has been optimized and by calculating a molecular structure that maximizes all energy while changing the molecular structure according to a calculation using a functional model higher in accuracy than the calculation (preliminary) ; a first substituent adding step (S7) of adding a substituent to the halfway TS (preliminary) when the halfway TS (preliminary) does not coincide with the original structure of the target TS (preliminary) at the target TS (preliminary) determining step (S6); a first substituent structural optimization calculating step (S8) of calculating optimization of a molecular structure of a substituent by calculating a molecular structure that minimizes all energy while changing the molecular structure with respect to only a site of the added substituent according to a calculation (preliminary) using a functional model lower in accuracy and then incorporating the resulting structure into the halfway TS (preliminary) optimization calculating step (S3) as a new halfway TS (preliminary); a first target TS determining step (S11) of determining whether the target TS obtained at the target TS optimization calculating step (S10) is a true target TS or is an original structure of the target TS by performing a vibration analysis of the obtained target TS by use of a vibration analytic function model; a halfway TS structural optimization calculating step (S14) of, when it is determined that the obtained target TS is the original structure of the target TS at the first target TS determining step (S11), calculating optimization of a molecular structure forming the halfway TS (preliminary) by performing a reading step (S13) of reading the halfway TS (preliminary) written onto the storage device at the writing step (S5), by fixing a reactive site of the halfway TS (preliminary), and by calculating the molecular structure that minimizes all energy while changing the molecular structure according to a calculation using a functional model higher in accuracy than the calculation (preliminary); a first halfway TS optimization calculating step (S15) of obtaining a halfway TS by unfixing the fixed reactive site of the halfway TS whose molecular structure has been optimized and by calculating the molecular structure that maximizes all energy while changing the molecular structure according to a calculation using a functional model higher in accuracy than the calculation (preliminary); a first halfway TS determining step (S16) of determining whether an obtained halfway TS is a true halfway TS or is an original structure of the halfway TS by performing a vibration analysis of the obtained halfway TS by use of a vibration analytic function model and, when it is determined that the obtained halfway TS is the original structure of the halfway TS, incorporating a halfway TS (preliminary) written prior to the halfway TS (preliminary) written onto the storage device into the reading step (S13); a second target TS determining step (S18) of, when it is determined (S17) that the obtained halfway TS is a true halfway TS at the first halfway TS determining step (S16), determining whether the halfway TS coincides with the target TS; a second substituent adding step (S19) of, when it is determined that the halfway TS does not coincide with the target TS at the second target TS determining step (S18), adding a substituent to the halfway TS; a second substituent structural optimization calculating step (S20) of calculating optimization of a molecular structure of a substituent by calculating a molecular structure that minimizes all energy while changing the molecular structure with respect to only a site of the added substituent according to a calculation using a functional model higher in accuracy than the calculation (preliminary); a second halfway TS optimization calculating step (S21) of obtaining a halfway TS by calculating a molecular structure that maximizes all energy while changing the molecular structure according to a calculation using a functional model higher in accuracy than the calculation (preliminary) while setting the halfway TS optimized at the second substituent structural optimization calculating step as a new halfway TS; and a second halfway TS determining step (S22) of determining whether the halfway TS obtained at the second halfway TS optimization calculating step (S21) is a true halfway TS or is an original structure of the halfway TS by performing a vibration analysis of the obtained halfway TS by use of a vibration analytic function model and, when it is determined that the obtained halfway TS is a true halfway TS, incorporating the halfway TS into the second target TS determining step (S18).

### Effects of the Invention

In the present invention, a halfway TS (preliminary) being in a stage prior to a target TS is found by use of a calculation (preliminary) lower in accuracy, and hence a calculation time to a halfway TS can be first shortened. Additionally, a target TS (preliminary) is found from the resulting halfway TS (preliminary) by use of a calculation (preliminary) lower in accuracy, for example, while adding a substituent, and a target TS is found by performing a calculation higher in accuracy for the first time after finding the target TS (preliminary), and therefore a calculation time can also be shortened here.
Additionally, if the target TS is not found from the target TS (preliminary) by performing a calculation higher in accuracy, the process temporarily returns to the halfway TS (preliminary), and then a halfway TS is found by performing a calculation higher in accuracy by use of the halfway TS (preliminary), and the target TS is found by use of a calculation higher in accuracy while adding a substituent or the like to the halfway TS. The process returns to the halfway TS (preliminary) in this way, and not the target TS (preliminary) but a halfway TS is found from the halfway TS (preliminary) this time by performing a calculation higher in accuracy, because a calculation time cannot be shortened if a calculation is performed from the beginning when the target TS cannot be obtained by use of a calculation higher in accuracy after finding the target TS (preliminary), and therefore an approach method differing from a previous calculation method can be performed, and the target TS can be found with a higher probability.
From the foregoing, a structure can be found substantially reliably while shortening a time even if it is a structure being in a transition state of a reaction product having a complex molecular structure.
Additionally, especially in the inventions recited in claims 1 to 3 and claims 6 to 9, when a calculation relative to TS optimization is performed, a reactive site is first fixed, and then a structure whose all energy becomes minimum is optimized, and then a calculation relative to optimization of a TS whose all energy becomes maximum is performed after unfixing the fixed reactive site, and therefore a calculation can be efficiently performed with high accuracy.
Especially, in the inventions recited in claims 2, 5, 7, and 9, the original structure of the halfway TS can be calculated by inputting data relative to a product, and therefore the target TS can be found more easily.

### BRIEF DESCRIPTION OF THE DRAWINGS

[FIG. 1] FIG. 1 is a schematic view of a chemical reaction transition state search system according to an embodiment of the present invention.
[FIG. 2] FIG. 2 is a flowchart showing data processing steps in the chemical reaction transition state search system, a chemical reaction transition state search method, and a chemical reaction transition state search program according to the present embodiment.
[FIG. 3] FIG. 3 is a chemical reaction formula showing a Curtius rearrangement reaction in which 2-(2-isocyanatophenylthio)thiophene is synthetically produced from 2-(thiophen-2-ylthio)benzoyl azide, and N₂ is emitted.
[FIG. 4] FIG. 4 is a chemical reaction formula selected to conceptualize a halfway TS before finding a target TS in the chemical reaction shown in FIG. 3.
[FIG. 5] FIG. 5 is a conceptual diagram showing the structure of an IG obtained by use of a halfway-TS original structure.
[FIG. 6] FIG. 6 is a graph showing calculation results in a CG-search calculating unit according to the present embodiment.
[FIG. 7] FIG. 7 is a conceptual diagram showing the structure of a CG (preliminary) obtained by the CG-search calculating unit according to the present embodiment.
[FIG. 8] FIG. 8 is a conceptual diagram showing the structure of a halfway TS (preliminary) obtained by a TS-structure-optimization calculating unit according to the present embodiment.
[FIG. 9] FIG. 9 is a conceptual diagram showing the structure of a methyl group.
[FIG. 10] FIG. 10 is a conceptual diagram showing the structure of a new halfway TS (preliminary) to which a substituent has been added by a substituent adding unit according to the present embodiment.
[FIG. 11] FIG. 11 is a chemical reaction formula including the halfway TS (preliminary) shown in FIG. 10.
[FIG. 12] FIG. 12 is a conceptual diagram showing the structure of a new halfway TS (preliminary).
[FIG. 13] FIG. 13 is a conceptual diagram showing the structure of a phenyl group.
[FIG. 14] FIG. 14 is a conceptual diagram showing the structure of a new halfway TS (preliminary) to which a phenyl group serving as a substituent has been added by the substituent adding unit according to the present embodiment.
[FIG. 15] FIG. 15 is a chemical reaction formula including the halfway TS (preliminary) shown in FIG. 14.
[FIG. 16] FIG. 16 is a conceptual diagram showing the structure of a new halfway TS (preliminary) obtained by the TS-structure-optimization calculating unit according to the present embodiment.
[FIG. 17] FIG. 17 is a conceptual diagram showing the structure of a thiophenethio group.
[FIG. 18] FIG. 18 is a chemical reaction formula including a new halfway TS (preliminary) to which a thiophenethio group serving as a substituent has been added by the substituent adding unit according to the present embodiment.
[FIG. 19] (a) is an output showing a result of a TS optimization calculation that is applied to a new halfway TS (preliminary) by the TS-structure-optimization calculating unit according to the present embodiment, and (b) is a conceptual diagram showing the structure of this new halfway TS (preliminary).
[FIG. 20] FIG. 20 is a conceptual diagram showing the structure of a new halfway TS (preliminary).
[FIG. 21] FIG. 21 is a conceptual diagram showing the structure of a halfway TS (preliminary) to which the substituent of the halfway TS (preliminary) of FIG. 20 has not yet been added.
[FIG. 22] FIG. 22 is a conceptual diagram showing the structure of a new halfway TS to which a substituent has been added by the substituent adding unit.
[FIG. 23] FIG. 23 is an output showing a calculation result relative to a halfway TS (target TS) obtained by performing a TS optimization calculation with respect to a halfway TS.
[FIG. 24] FIG. 24 is a conceptual diagram showing the structure of a halfway TS (target TS) obtained by performing a TS optimization calculation.
[FIG. 25] (a) is a flowchart showing a general flow from an initial geometry (IG) to the calculation of a transition-state geometry (TS) through a candidate geometry (CG) of a transition-state geometry, and (b) is a flowchart showing a flow in which a target TS (preliminary) is analyzed from an IG by use of a calculation (preliminary), and then an optimization calculation is performed, and a target TS is found, and (c) is a flowchart showing a flow in which a halfway TS, not a target TS, is temporarily analyzed from an IG, and then a target TS is analyzed and found.
[FIG. 26] FIG. 26 is a flowchart showing a conventional technique of a search method for searching for a transition state that is the most basic one.
[FIG. 27] FIG. 27 is a flowchart showing a conventional technique of a search method for searching for a transition state.
[FIG. 28] FIG. 28 is a flowchart showing a conventional technique of a search method for searching for a transition state. Description of Signs

- 1: Chemical reaction transition state search system
- 2: Input device
- 3: Arithmetic processing unit
- 4: Output device
- 5: Storage device
- 6: Data input unit
- 7: IG forming unit
- 8: CG-search calculating unit
- 9: TS-structure-optimization calculating unit
- 10: Reactive-site fixing unit
- 11: Substituent adding unit
- 12: Structural optimization calculating unit
- 13: Data output unit
- 14: Halfway-TS setting unit
- 20: Calculation model
- 21: Calculation (preliminary) model
- 22: Vibration analytic function model
- 25: IG data
- 26: CG data
- 27: Substituent data
- 28: Reactive site data
- 29: Halfway-TS-(preliminary) historical data
- 30: Halfway TS data
- 31: Target TS data
- 41: 2-(thiophen-2-ylthio)benzoyl azide
- 42: 2- (2-isocyanatophenylthio) thiophene
- 43: Nitrogen
- 44: Formyl azide
- 45: Isocyanic acid
- 46: Nitrogen
- 47: IG
- 48: CG (preliminary)
- 49, 49a, 49b, 49c: Halfway TS (preliminary)
- 50: Methyl group
- 51: Phenyl group
- 52: Thiophenethio group
- 53: Halfway TS
- 54: Target TS

### BEST MODE FOR CARRYING OUT THE INVENTION

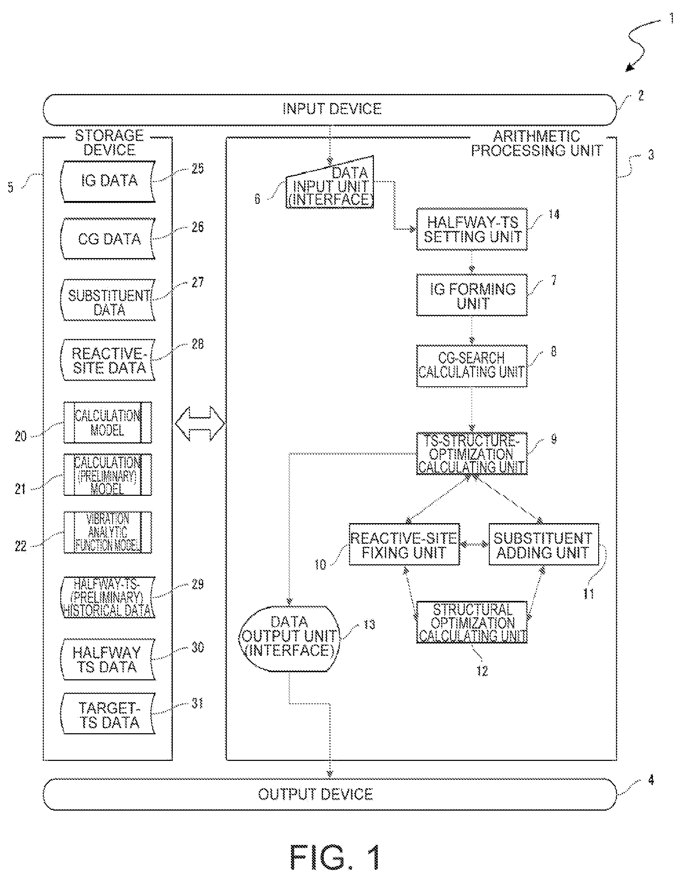

A chemical reaction transition state search system, a chemical reaction transition state search method, and a chemical reaction transition state search program will be hereinafter described with reference to FIG. 1 to FIG. 24 according to a most preferred embodiment of the present invention.
FIG. 1 is a schematic view of a chemical reaction transition state search system 1 according to the present embodiment. In FIG. 1, the chemical reaction transition state search system 1 according to the present embodiment is composed of an input device 2, an arithmetic processing unit 3, an output device 4, and a storage device 5.
First, various data is input from the input device 2, and the data is processed in the arithmetic processing unit 3, and is stored in the storage device (data base) 5 that realizes temporary storage and permanent storage. The data stored therein is also read and processed by each component of the arithmetic processing unit 3. Data obtained by being processed by the arithmetic processing unit 3 is displayed by the output device 4 or is output to an external device.
Specific examples of the input device 2 include a keyboard, a mouse, a pen tablet, an optical or magnetic reader, a receiver that receives data from an analyzer or from a measuring device, such as a computer, through a communication line, or a combination of these devices.
The arithmetic processing unit 3 is composed of a data input unit 6, an IG forming unit 7, a CG-search calculating unit 8, a TS-structure-optimization calculating unit 9, a reactive-site fixing unit 10, a substituent adding unit 11, a structural optimization calculating unit 12, a data output unit 13, and a halfway-TS setting unit 14.
The storage device 5 is capable of storing a calculation model 20, a calculation (preliminary) model 21, a vibration analytic function model 22, IG data 25, CG data 26, substituent data 27, reactive-site data 28, halfway-TS-(preliminary) historical data 29, halfway TS data 30, and target-TS data 31.
A display, such as a CRT display, a liquid crystal display, a plasma display, or an organic EL display, or a display device of, for example, a printer, or a signal emitting device, such as a transmitter, used to transmit signals to an external device can be mentioned as a specific example of the output device 4.
The data input unit 6 functions as an interface with the input device 2, and the data output unit 13 functions as an interface with the output device 4.
The output device 4 can display data relative to the calculation model 20, the calculation (preliminary) model 21, and the vibration analytic function model 22 stored in the storage device 5, can display data, such as the IG data 25, the CG data 26, the substituent data 27, the reactive-site data 28, the halfway-TS-(preliminary) historical data 29, the halfway TS data 30, and the target-TS data 31, and can display a calculation process or calculation results of the arithmetic processing unit 3 by means of the data output unit 13. Furthermore, the output device 4 can transmit these data and results to external devices.

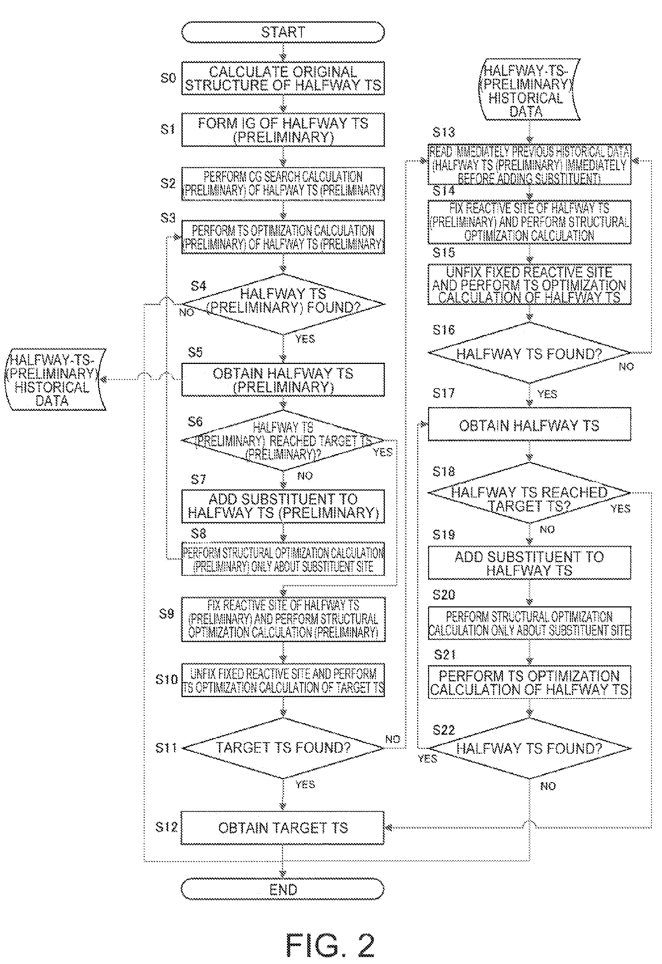

Next, the function of the arithmetic processing unit 3 and the flow of data processed by the arithmetic processing unit 3 will be described with reference to FIG. 2 to FIG. 24 in addition to FIG. 1.
FIG. 2 is a flowchart showing data-processing steps in the chemical reaction transition state search system, the chemical reaction transition state search method, and the chemical reaction transition state search program according to the present embodiment. In the present embodiment, a description will be given while calculating a transition state (TS) relative to a Curtius rearrangement reaction to synthetically produce 2-(2-isocyanatophenylthio)thiophene. In the following description, steps shown in FIG. 2 are placed within parentheses.

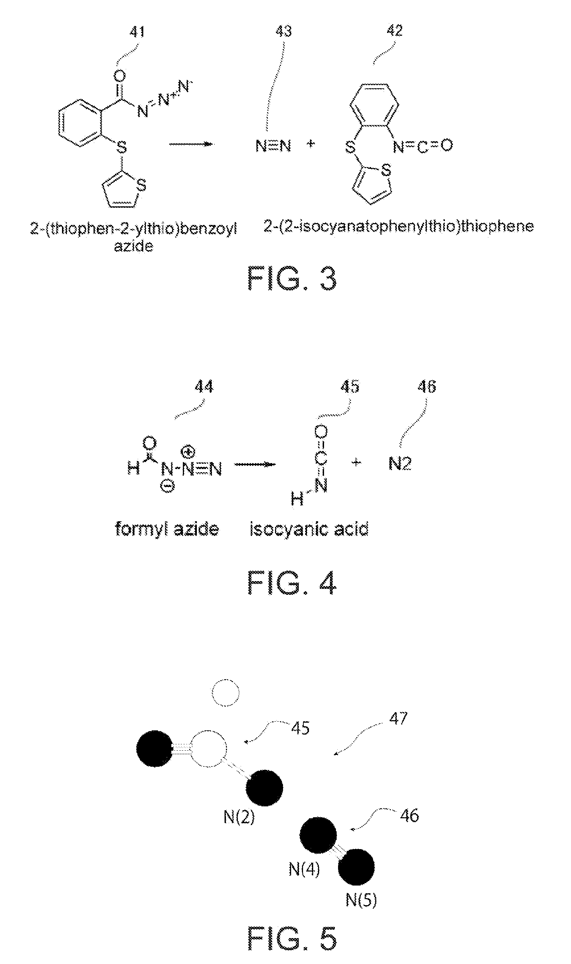

FIG. 3 is a chemical formula showing a Curtius rearrangement reaction in which 2-(2-isocyanatophenylthio)thiophene 42 is synthetically produced from 2- (thiophen-2-ylthio)benzoyl azide 41 and in which N₂ (nitrogen) 43 is emitted. FIG. 4 is a chemical reaction formula selected to conceptualize a halfway TS before finding a target TS in the chemical reaction shown in FIG. 3. Specifically, isocyanic acid 45 and N₂ (nitrogen) 46 are generated from formyl azide 44 that is a reactant. The TS in this chemical formula is set as a halfway TS.
In setting the halfway TS, data (not shown) relative to reaction products is input from the input device 2 in FIG. 1. The reaction-product data is input to the halfway-TS setting unit 14 through the data input unit 6 of the arithmetic processing unit 3. In the halfway-TS setting unit 14, the original structure of the halfway TS is calculated by reading the substituent data 27 stored in the storage device 5 and by removing substituents included in the reaction-product data (step S0). Alternatively, the halfway-TS setting unit 14 may calculate the halfway-TS original structure in such a manner as to read the reactive-site data 28 included in the storage device 5 and to leave only reactive sites included in the reaction-product data (step S0). At least one substituent may be removed, or a plurality of substituents may be removed if these substituents can be removed. No limitations are imposed on the number of reactive sites to be left as long as one or more reactive sites are left.
Although the original structure of the halfway TS is calculated by providing the halfway-TS setting unit 14 in the present embodiment, the original structure of the halfway TS may be beforehand input from the input device 2 without providing the halfway-TS setting unit 14. In this case, step S0 of FIG. 2 can be omitted.

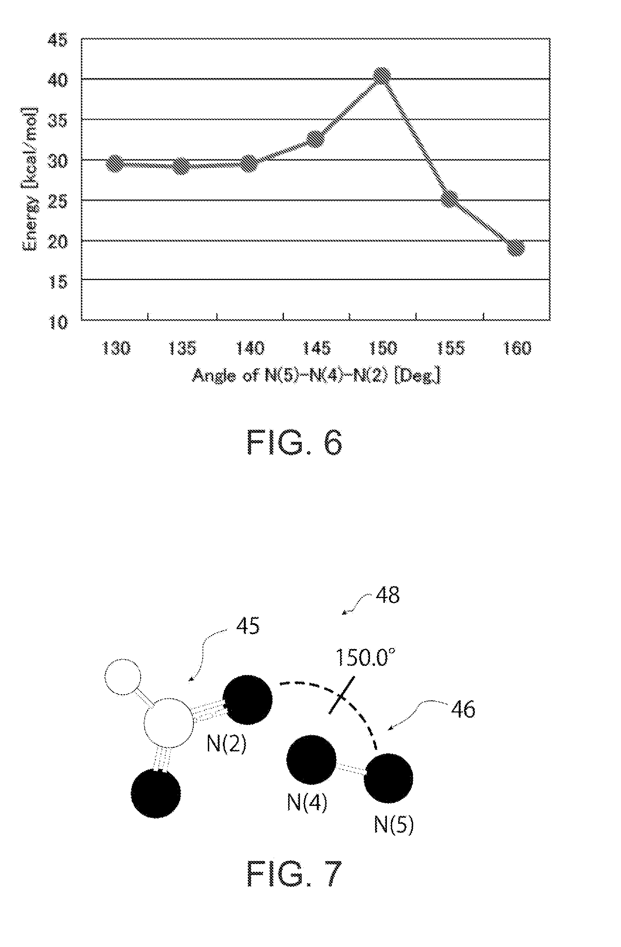

In this patent application, although the original structure of the halfway TS is the same in the kind and in the number of atoms that are structural components as the halfway TS, the original structure of the halfway TS has not yet been optimized, and hence is not identical with the halfway TS. In other words, the halfway TS and the halfway-TS original structure differ from each other, and are used as different ones. The same applies to other original structures.
Thereafter, the IG forming unit 7 forms an IG to find a halfway TS by use of a halfway-TS original structure calculated by the halfway-TS setting unit 14 (step S1). To form this IG, an intermediate structure with respect to formyl azide 44 that is a reactant is calculated by use of the halfway-TS original structure calculated by the halfway-TS setting unit 14. The intermediate structure is calculated by an arithmetic mean of internal coordinates of a molecular structure (i.e., coordinates defined by use of interatomic distances, interatomic angles, and dihedral angles). For example, the intermediate structure between a molecule in which the interatomic distance between atoms C-H is 1.5 Å (angstrom) and a molecule in which the interatomic distance between atoms C-H is 3.0 Å can be represented as a molecule in which the interatomic distance between atoms C-H is 2.25 Å.
An IG obtained in this way is shown in FIG. 5. FIG. 5 is a structural drawing showing an IG 47 obtained by use of a halfway-TS original structure. The IG 47 formed here is stored in the storage device 5 by the IG forming unit 7 so as to be read as IG data 25.
If the halfway-TS setting unit 14 is not provided unlike the above-mentioned structure, a halfway-TS original structure input from the input device 2 is input to the IG forming unit 7 so as to find an IG.
In the CG-search calculating unit 8, a CG search calculation (preliminary) is performed by use of the formed IG 47. This calculation (preliminary) denotes a calculation lower in accuracy than a "mere calculation" as already described, and, in general, is shorter in calculation time than the mere calculation. Additionally, a CG or a TS obtained by performing this calculation (preliminary) is inscribed as a CG (preliminary) or a TS (preliminary), and is distinguished from a CG or a TS obtained by the mere calculation.
In the CG-search calculating unit 8, a calculation (preliminary) model 21 using the minimum energy path method is read from the storage device 5, and then the angle among nitrogen atoms N(5)-N(4)-N(2) of the IG of FIG. 5 is changed by six stages from 130° to 160° by use of the calculation (preliminary) model 21, and all energy during each period is calculated (step S2). Calculation results are shown in FIG. 6.

The abscissa axis of a graph shown in FIG. 6 represents an angle between nitrogen atoms, and the ordinate axis represents all energy. As is apparent from the calculation results of FIG. 6, all energy is highest when the angle is 150°, and therefore this is assumed as a CG (preliminary). The structure of this CG (preliminary) is shown in FIG. 7.
In FIG. 7, the CG 48 has an angle between nitrogen atoms of 150°. CG data 26 relative to this CG (preliminary) 48 is readably stored in the storage device 5. The CG-search calculating unit 8 employs a model using the minimum energy path method as the calculation (preliminary) model 21 in the present embodiment. However, without being limited to this, a calculation (preliminary) model 21 using, for example, the saddle method or the contour method may be employed if a CG search calculation can be performed.

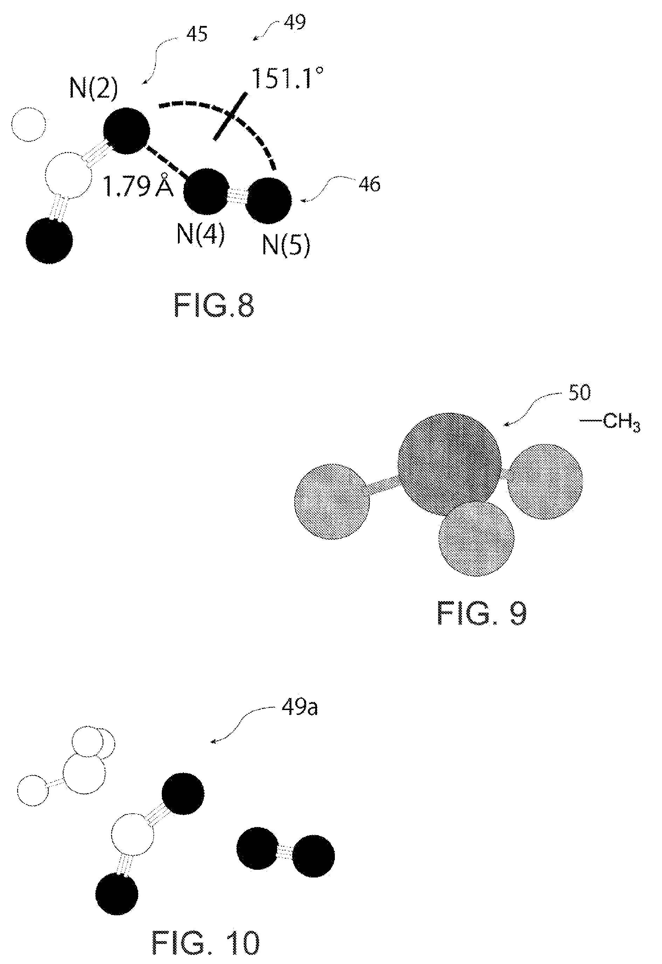

Thereafter, the TS-structure-optimization calculating unit 9 performs an optimization calculation by use of a CG (preliminary) 48 obtained in the CG-search calculating unit 8 (step S3). In more detail, the TS-structure-optimization calculating unit 9 reads a calculation (preliminary) model 21 used to perform an optimization calculation from the storage device 5, and all energy of a structure is calculated by use of this calculation (preliminary) model 21. A structure whose all energy reaches a maximum value is found while further performing a calculation. When the structure whose all energy reaches a maximum value is obtained, the TS-structure-optimization calculating unit 9 determines that this is a halfway TS (preliminary) (steps S4 and S5).
After determining that this is a halfway TS (preliminary), the TS-structure-optimization calculating unit 9 reads a vibration analytic function model 22 from the storage device 5, then performs a vibration analysis, and determines whether this halfway TS (preliminary) 49 is a true halfway TS (preliminary) 49 or this halfway TS (preliminary) 49 is merely an original structure of the halfway TS (preliminary). This determination is made by whether normal vibrations having imaginary frequency solely exist when the vibration analysis is performed. If a halfway TS (preliminary) cannot be obtained at step S4, the calculation performance is ended without proceeding to another step.
The halfway TS (preliminary) obtained at steps S4 and S5 is shown in FIG. 8. The angle between nitrogen atoms was 150° in the CG (preliminary) 48 of FIG. 7, whereas the angle between nitrogen atoms of the halfway TS (preliminary) 49 was 151.1° as a result of an optimization calculation performed by use of the calculation (preliminary) model 21. After obtaining the halfway TS (preliminary) 49, the TS-structure-optimization calculating unit 9 readably stores data relative to this halfway TS (preliminary) 49 in the storage device 5 as halfway-TS-(preliminary) historical data 29 (step S5). The meaning of being stored as halfway-TS-(preliminary) historical data 29 shows the fact that, whenever step S5 mentioned above is performed, data relative to the halfway TS (preliminary) 49 is stored while storing the order in which step S5 is performed. In other words, data relative to the halfway TS (preliminary) 49 and an ordinal number that is sequentially increased from "1st" are stored as halfway-TS-(preliminary) historical data 29.

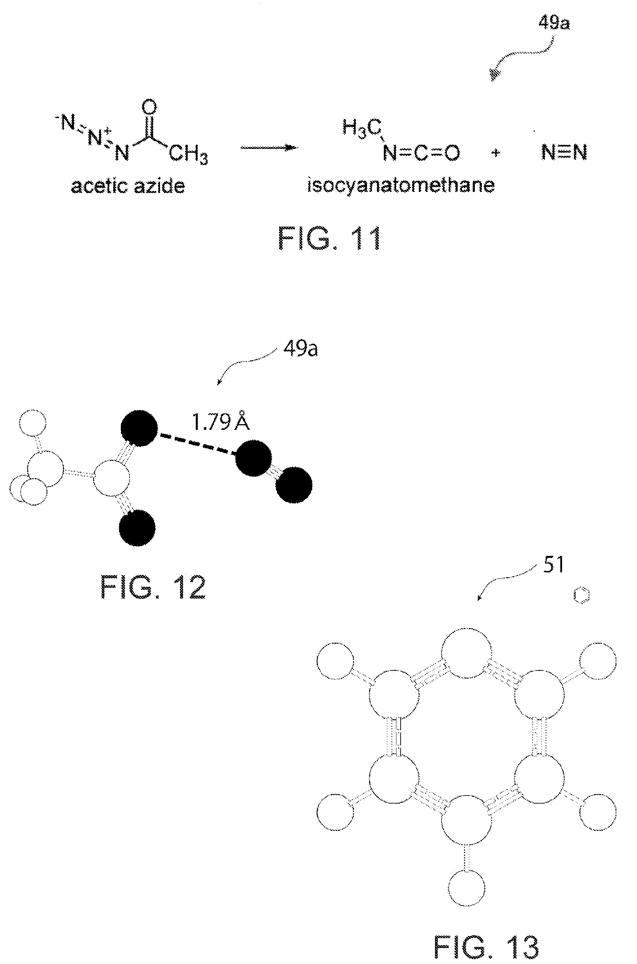

Thereafter, the TS-structure-optimization calculating unit 9 determines whether the halfway TS (preliminary) 49 obtained above coincides with the original structure of the target TS (preliminary) (step S6). In a case in which a reaction product is input from the input device 2, the determination of coincidence therebetween is included in data relative to the reaction product, and therefore a target-TS original structure included in the reaction product is stored in the storage device 5. In a case in which a halfway-TS original structure is input from the input device 2, it is necessary to separately pre-input and pre-store the target-TS original structure in the storage device 5. Thereafter, the target-TS original structure is read from the storage device 5 by the TS-structure-optimization calculating unit 9, and then this is compared with the halfway TS (preliminary) 49, and it is determined whether an original structure to be regarded as having already been included in the target TS is included in the halfway TS (preliminary) 49.
If it is determined that the halfway TS (preliminary) 49 does not yet coincide with the original structure of the target TS (preliminary), the substituent adding unit 11 reads substituent data 27 from the storage device 5, and a calculation to add a substituent to this halfway TS (preliminary) 49 is performed (step S7). A methyl group 50 shown in FIG. 9 can be mentioned as an example of this substituent. The substituent adding unit 11 operates to add substituent data 27 relative to the methyl group 50 read from the storage device 5 in such a manner as to be exchanged for hydrogen atoms included in the halfway TS (preliminary) 49. A combination of a substituent and atoms that are exchanged to add the substituent is known beforehand, and therefore it is recommended to include this combination in the substituent data 27. In other words, for example, both data relative to the methyl group 50 and data relative to hydrogen atoms that are exchanged for the methyl group 50 are included in the substituent data 27, and a search is made for hydrogen atoms of the halfway TS (preliminary) 49 that coincide with data relative to these hydrogen atoms, and then the hydrogen atoms are exchanged for the methyl group 50.
A new halfway TS (preliminary) to which a substituent has been added by the substituent adding unit 11 in this way is shown in FIG. 10, and a reaction including this new halfway TS (preliminary) 49a is shown in FIG. 11. This new halfway TS (preliminary) 49a is subjected to the performance of the calculation of structural optimization by the structural optimization calculating unit 12 only about the newly added substituent (step S8).

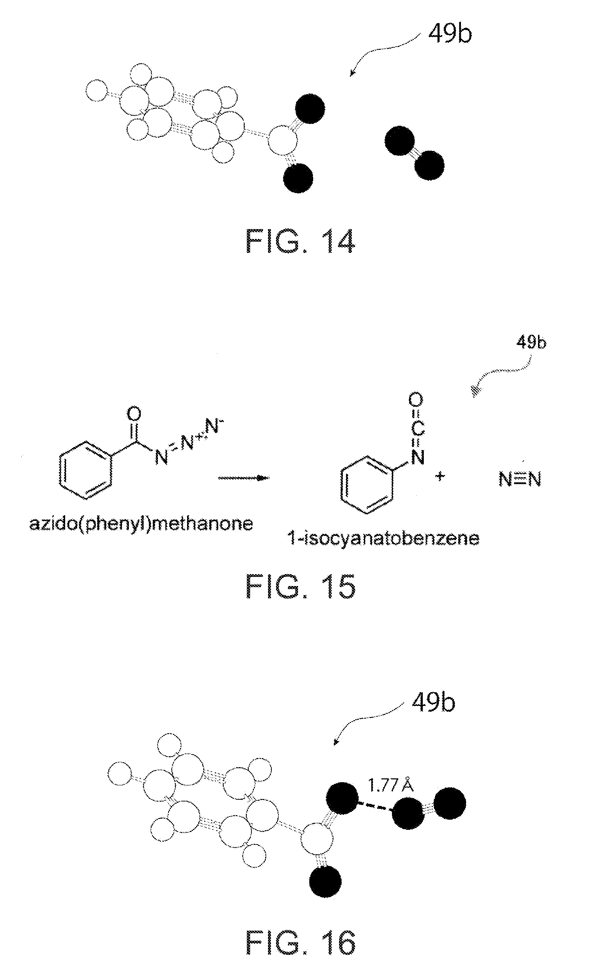

A structural optimization calculation only about the methyl group 50 shown in FIG. 9 is performed in the structural optimization calculating unit 12, and, in this case, a calculation (preliminary) model 21 relative to structural optimization is read from the storage device 5, and a structural all-energy calculation is performed by use of this model, and a structure whose all energy reaches a minimum value is found. Thereafter, a new halfway TS (preliminary) 49a is again subjected to a TS optimization calculation by use of the TS-structure-optimization calculating unit 9 (step S3), and it is determined whether the halfway TS (preliminary) 49a has been found (step S4). If the halfway TS (preliminary) 49a has been obtained, this is stored in the storage device 5 as halfway-TS- (preliminary) historical data 29 (step S5), and it is again determined whether the halfway TS (preliminary) 49a coincides with the original structure of the target TS (preliminary) (step S6). If the halfway TS (preliminary) 49a does not coincide therewith, steps S7, S8, and S3 to S6 of FIG. 2 are repeatedly performed. In the present embodiment, the TS optimization calculation of step S3 using the methyl group 50, which was a first added substituent, was performed, and then the halfway TS (preliminary) 49a (whose structure is shown in FIG. 12) was obtained, and new data of the halfway TS (preliminary) 49a with respect to preceding data of the halfway TS (preliminary) 49 was stored in the storage device 5 as halfway-TS-(preliminary) historical data 29 to which the ordinal number "2nd" was newly added in this case. As shown in FIG. 12, the distance between nitrogen atoms of the halfway TS (preliminary) 49a was 1.79 angstroms. The following process is recommended to add an ordinal number. In this process, the calculating formula n=n+1 is built into step S5, and the equation n=0 is set as an initial value, and, on the other hand, "n" that is increased one by one from 1 is added to data relative to a halfway TS (preliminary) that is first stored in a one-by-one increment manner at step S5, and the resulting data is stored as halfway-TS-(preliminary) historical data 29. However, the new halfway TS (preliminary) has not yet reached the original structure of the target TS (preliminary) at step S6, and therefore a substituent is again added by the substituent adding unit 11. This substituent is added in the same way as described above. The substituent added thereto is a phenyl group 51, and this structure is as shown in FIG. 13. The new halfway TS (preliminary) 49b to which the phenyl group 51 has been added as a substituent by the substituent adding unit 11 is shown in FIG. 14, and a reaction including this new halfway TS (preliminary) 49b is shown in FIG. 15. The new halfway TS (preliminary) 49b is subjected to the performance of the calculation of structural optimization by the structural optimization calculating unit 12 only about the newly added substituent (step S8).

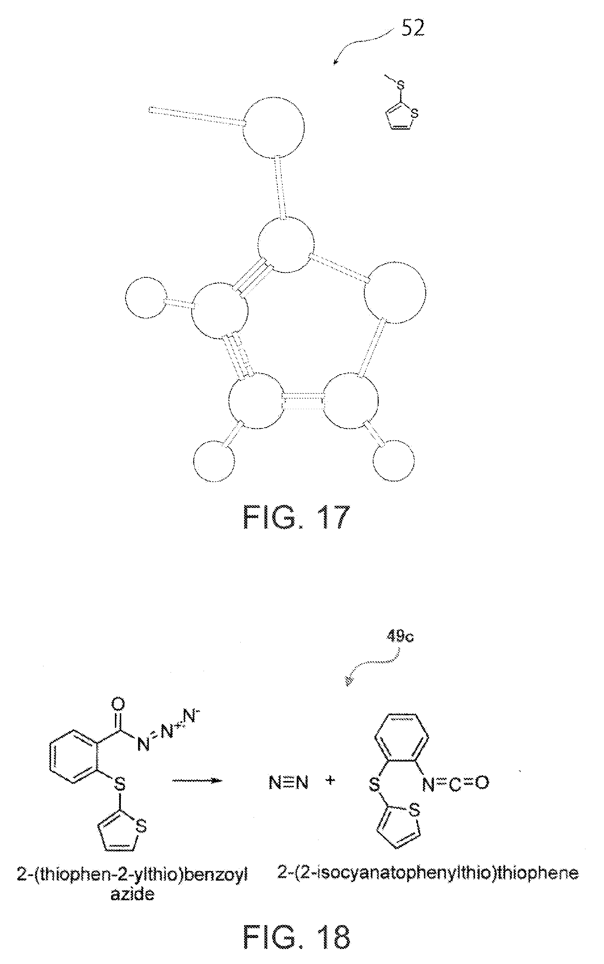

A structural optimization calculation only about the phenyl group 51 shown in FIG. 13 is performed in the structural optimization calculating unit 12, and, in this case, a calculation (preliminary) model 21 relative to structural optimization is read from the storage device 5, and then a structural all-energy calculation is performed by use of this model, and a structure whose all energy reaches a minimum value is found.
Thereafter, a new halfway TS (preliminary) 49b is again subjected to a TS optimization calculation by the TS-structure-optimization calculating unit 9 (step S3), and it is determined whether the halfway TS (preliminary) 49b has been found (step S4), and, if found, this halfway TS (preliminary) 49b and an ordinal number (3rd) are stored in the storage device 5 as halfway-TS-(preliminary) historical data 29 (step S5), and it is again determined whether the halfway TS (preliminary) 49b coincides with the original structure of the target TS (preliminary) (step S6). In the present embodiment, the fact that the halfway TS (preliminary) 49b has been obtained was determined at step S4, and this halfway TS (preliminary) 49b was readably stored in the storage device 5 as halfway-TS-(preliminary) historical data 29. The halfway TS (preliminary) 49b obtained here is shown in FIG. 16. As shown in FIG. 16, the distance between nitrogen atoms of the halfway TS (preliminary) 49b was 1.77 angstroms.
However, even the new halfway TS (preliminary) 49b has not yet reached the original structure of the target TS (preliminary) at step S6, and therefore a substituent is again added by the substituent adding unit 11. This substituent is added in the same way as described above. The substituent added thereto is a thiophenethio group 52. Its structure is as shown in FIG. 17.
A reaction including a new halfway TS (preliminary) 49c to which the thiophenethio group 52 has been added as a substituent by the substituent adding unit 11 is shown in FIG. 18. This new halfway TS (preliminary) 49c is subjected to the performance of the calculation of structural optimization by the structural optimization calculating unit 12 only about the newly added substituent (step S8).

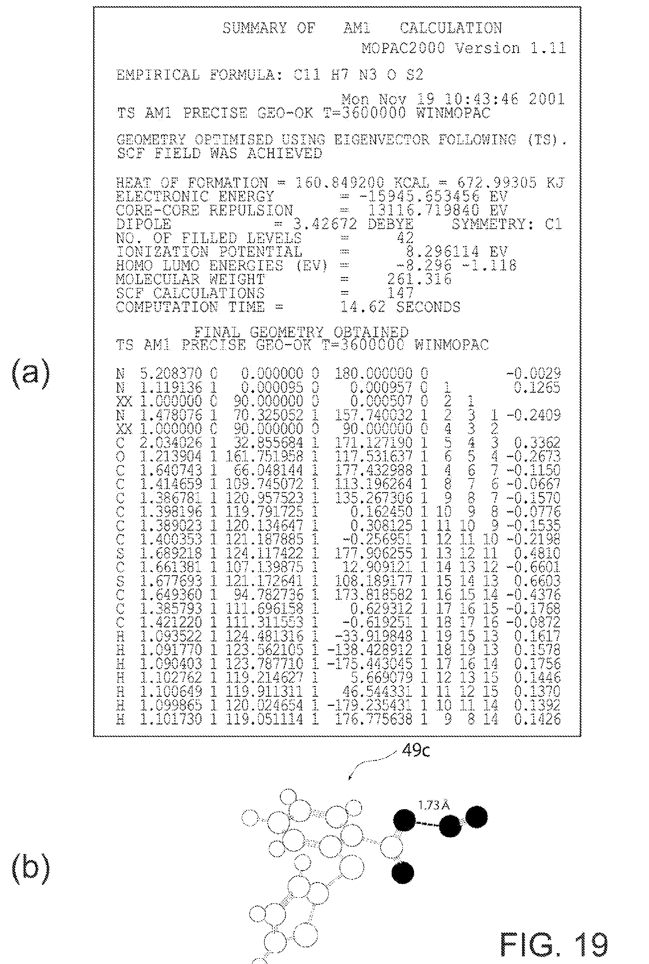

A structural optimization calculation only about the thiophenethio group 52 shown in FIG. 17 is performed in the structural optimization calculating unit 12, and, in this case, a calculation (preliminary) model 21 relative to structural optimization is read from the storage device 5, and then a structural all-energy calculation is performed by use of this model, and a structure whose all energy reaches a minimum value is found.
Thereafter, a new halfway TS (preliminary) 49c is again subjected to a TS optimization calculation by the TS-structure-optimization calculating unit 9 (step S3), and it is determined whether the halfway TS (preliminary) 49c has been found (step S4), and, if found, this halfway TS (preliminary) 49c is stored in the storage device 5 as halfway-TS- (preliminary) historical data 29 (step S5), and it is again determined whether the halfway TS (preliminary) 49c coincides with the original structure of the target TS (preliminary) (step S6). In the present embodiment, the fact that the halfway TS (preliminary) 49c has been obtained was determined at step S4, and this halfway TS (preliminary) 49c and an ordinal number (4th) were readably stored in the storage device 5 as halfway-TS-(preliminary) historical data 29.
The halfway TS (preliminary) 49c obtained this time coincides with the original structure of the target TS (preliminary), and calculation results of this are shown in (a) of FIG. 19, and the structure of the halfway TS (preliminary) 49c is shown in (b) of FIG. 19. In (a) of FIG. 19, pieces of information about a calculation title, a date, etc., are output at the upper part of the figure, and calculation results of energy, calculation time, etc., are output at the middle part of the figure, and kinds of atoms and internal coordinates thereof are output at the lower part of the figure. All of these results are calculation results obtained according to the non-empirical molecular orbital calculation method (AM1 method).

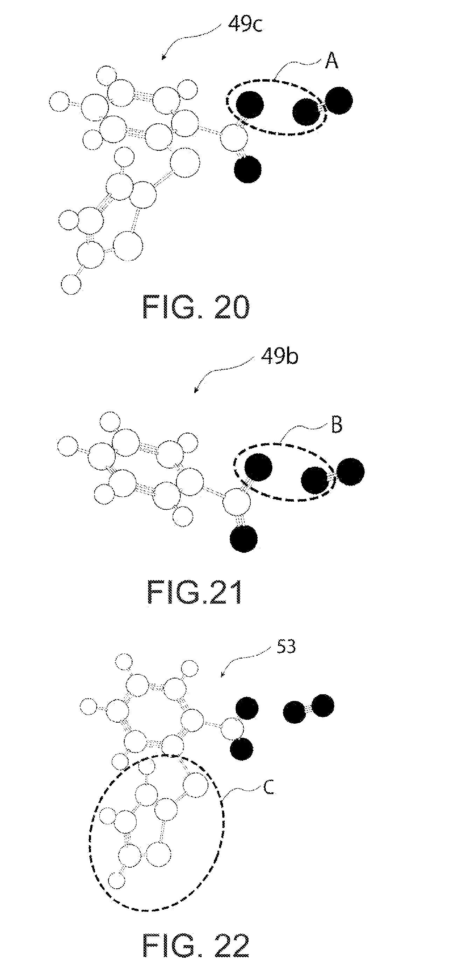

If the TS-structure-optimization calculating unit 9 determines that the halfway TS (preliminary) 49c coincides with the original structure of the target TS (preliminary), the reactive-site fixing unit 10 fixes the reactive site of the halfway TS (preliminary) 49c, and a structural optimization calculation is performed. In detail, the reactive-site fixing unit 10 reads reactive-site data 28 from the storage device 5, and data relative to a reactive site included in the halfway TS (preliminary) 49c is obtained from the reactive-site data 28 by searching, and then this reactive site is fixed. A portion fixed as the reactive site is designated by reference numeral "A" enclosed by an ellipse of the broken line as shown in FIG. 20. Furthermore, the structural optimization calculating unit 12 reads the calculation (preliminary) model 21 to perform a structural optimization calculation from the storage device 5, and then calculates structural all energy by use of this model, and calculates and finds a structure whose all energy reaches a minimum value (step S9).

Furthermore, the reactive site previously fixed by the reactive-site fixing unit 10 is unfixed, and the TS-structure-optimization calculating unit 9 allows the halfway TS (preliminary) 49c, which has already undergone a structural optimization calculation and which is substantially a target TS (preliminary) because the target TS (preliminary) and the original structure have already coincided with each other, to undergo a TS optimization calculation. In detail, the TS-structure-optimization calculating unit 9 reads a calculation model 20 used for a TS optimization calculation from the storage device 5, and performs a structural all-energy calculation by use of this calculation model 20 (step S10). Furthermore, while performing the calculation, a structure whose all energy reaches a maximum value is found. If the structure having the maximum value is obtained, the TS-structure-optimization calculating unit 9 determines that this structure is a target TS (steps S11 and S12).
After determining that the structure is a target TS, the TS-structure-optimization calculating unit 9 further reads a vibration analytic function model 22 from the storage device 5, and performs a vibration analysis, and determines whether this target TS is a true target TS or is merely the original structure of the target TS. This determination is made by whether normal vibrations having imaginary frequency solely exist when the vibration analysis is performed (steps S11 and S12).
If the true target TS is obtained in this way, the calculation performance is ended.

On the other hand, the target TS was not able to be obtained at step S11 in the present embodiment, and hence the process proceeds from step S11 to step S13 of FIG. 2. The target TS was not able to be obtained at step S13, and hence the reactive-site fixing unit 10 or the structural optimization calculating unit 12 reads a halfway TS (preliminary) to which the present substituent has not yet been added from the halfway-TS-(preliminary) historical data 29 stored in the storage device 5 (step S13). In the present embodiment, what is read therefrom precedes the halfway TS (preliminary) 49c, and hence is the halfway TS (preliminary) 49b shown in FIG. 14. This reading operation is performed by selecting the one that is immediately previous to the halfway TS (preliminary) whose ordinal number added thereto is the greatest among a plurality of halfway TS (preliminary) in the halfway-TS-(preliminary) historical data 29. In this embodiment, an increment in ordinal numbers has been made to "4th," and hence a return is made from the ordinal number "4th" to the immediately previous ordinal number "3rd," and the halfway TS (preliminary) having the ordinal number 3rd is selected and read.
The reactive-site fixing unit 10 fixes the reactive site of this halfway TS (preliminary) 49b, and the structural optimization calculating unit 12 performs the structural optimization calculation of the halfway TS (preliminary) 49b. In detail, the reactive-site fixing unit 10 reads reactive-site data 28 from the storage device 5, and then searches for and obtains data relative to the reactive site included in the halfway TS (preliminary) 49b from this reactive-site data 28, and fixes the thus obtained reactive site. A portion fixed as the reactive site is designated by reference numeral "B" enclosed by an ellipse of the broken line as shown in FIG. 21. Furthermore, the structural optimization calculating unit 12 reads the calculation model 20 to perform a structural optimization calculation from the storage device 5, and then calculates structural all energy by use of this model, and calculates and finds a structure whose all energy reaches a minimum value (step S14).
Furthermore, the reactive-site fixing unit 10 unfixes the previously fixed reactive site, and the TS-structure-optimization calculating unit 9 performs a TS optimization calculation with respect to the halfway TS that has already undergone a structural optimization calculation. In detail, the TS-structure-optimization calculating unit 9 reads a calculation model 20 used for a TS optimization calculation from the storage device 5, and performs a structural all-energy calculation by use of this calculation model 20 (step S15). Furthermore, while performing the calculation, a structure whose all energy reaches a maximum value is found. If the structure having the maximum value is obtained, the TS-structure-optimization calculating unit 9 determines that this structure is a halfway TS (steps S16 and S17) .
If it is determined at step S16 that this is not a halfway TS, the process returns to step S13 again, and the reactive-site fixing unit 10 or the structural optimization calculating unit 12 reads a halfway TS (preliminary) to which the present substituent has not yet been added from the halfway-TS- (preliminary) historical data 29 stored in the storage device 5 (step S13). In other words, a halfway TS (preliminary) having the immediately previous ordinal number is here selected and read, and step S14 and steps following this step are performed. Data relative to the halfway TS (preliminary) is not shown in FIG. 2 and is stored in the halfway-TS- (preliminary) historical data 29 along with its ordinal number, and it is preferable to perform calculations of steps S14 and S15 by use of halfway TS (preliminary) data whose ordinal number is 1st because the ordinal number 1st is the last ordinal number and is preferable to end the calculation operation if a halfway TS is not found at step S16.

After determining that the structure is a halfway TS, the TS-structure-optimization calculating unit 9 further reads a vibration analytic function model 22 from the storage device 5, and performs a vibration analysis, and determines whether this halfway TS is a true halfway TS or is merely the original structure of the halfway TS. This determination is made by whether normal vibrations having imaginary frequency solely exist when the vibration analysis is performed (steps S16 and S17). Data relative to the true halfway TS obtained by the TS-structure-optimization calculating unit 9 is readably stored in the storage device 5 by the TS-structure-optimization calculating unit 9 as halfway TS data 30.
After finding the halfway TS at steps S16 and S17, it is determined at step S18 whether this halfway TS reaches the original structure of the target TS, i.e., whether this halfway TS coincides therewith.
Ground for determination of whether the halfway TS coincides therewith is included in data relative to a reaction product if the reaction product is input from the input device 2 as described above, and therefore a target-TS original structure included in the reaction product is beforehand stored in the storage device 5. If a halfway-TS original structure is input from the input device 2, it is necessary to beforehand input and store the target-TS original structure separately in the storage device 5. The target-TS original structure is read from the storage device 5 by the TS-structure-optimization calculating unit 9, and is compared with the halfway TS, and it is determined whether an original structure, which should be included in the target TS, has been already included in the halfway TS.

If it is determined that the halfway TS has not yet coincided with the original structure of the target TS, the substituent adding unit 11 reads substituent data 27 from the storage device 5, and performs a calculation to add a substituent to this halfway TS (step S19). A thiophenethio group 52 enclosed by the ellipse of the broken line designated by reference numeral C in FIG. 22 is selected as this substituent. The target TS (preliminary) has already been obtained at step S6, and the substituent to be selected from a relationship with this structure is selected unambiguously.
The substituent adding unit 11 operates to add substituent data 27 relative to the thiophenethio group 52 read from the storage device 5 in such a manner as to be exchanged for hydrogen atoms included in the halfway TS 53.
A new halfway TS 53 to which a substituent has been added by the substituent adding unit 11 in this way is shown in FIG. 22. This new halfway TS 53 is subjected to the performance of the calculation of structural optimization by the structural optimization calculating unit 12 only about the newly added substituent (step S20). A structural optimization calculation only about the thiophenethio group 52 designated by reference numeral C in FIG. 22 is performed in the structural optimization calculating unit 12, and, in this case, a calculation model 20 relative to structural optimization is read from the storage device 5, and a structural energy calculation is performed by use of this model, and a structure whose all energy reaches a minimum value is found.
The TS-structure-optimization calculating unit 9 further performs a TS optimization calculation with respect to the halfway TS 53 that has already undergone the structural optimization calculation. In detail, the TS-structure-optimization calculating unit 9 reads a calculation model 20 used for a TS optimization calculation from the storage device 5, and performs a structural all-energy calculation by use of this calculation model 20 (step S21). Furthermore, while performing the calculation, a structure whose all energy reaches a maximum value is found. If the structure having the maximum value is obtained, the TS-structure-optimization calculating unit 9 determines that this structure is a halfway TS (steps S22 and S17).
If it is determined that the halfway TS has not been found at step S22, the calculation performance is ended.

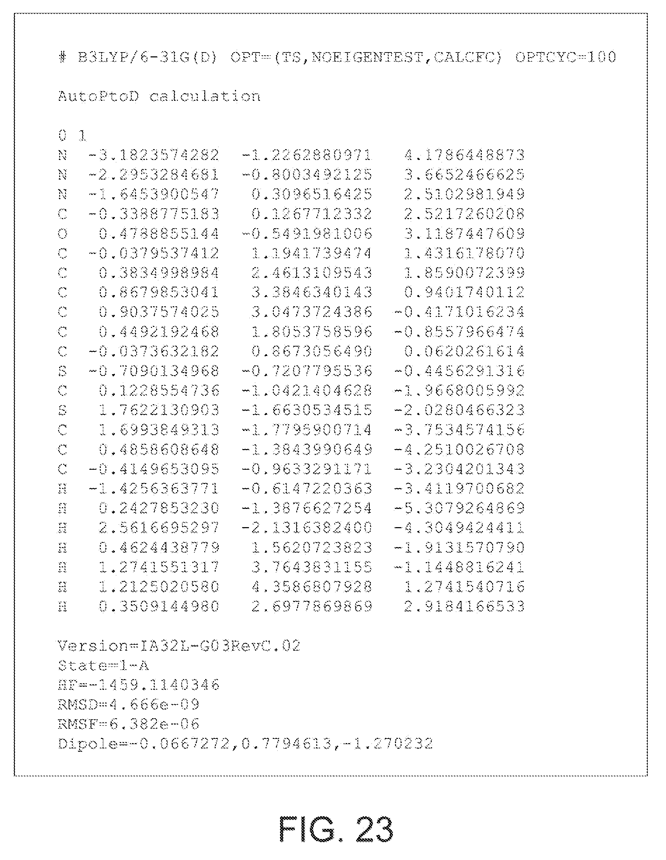

If it is determined that the structure is a halfway TS, the TS-structure-optimization calculating unit 9 further reads a vibration analytic function model 22 from the storage device 5, and performs a vibration analysis, and determines whether this halfway TS is a true halfway TS or is merely the original structure of the halfway TS. This determination is made by whether normal vibrations having imaginary frequency solely exist when the vibration analysis is performed (step S17).
After finding the halfway TS at step S17, it is determined at step S18 whether this halfway TS reaches the original structure of the target TS, i.e., whether this halfway TS coincides therewith. If the halfway TS reaches the original structure of the target TS, the calculation performance is ended.
Calculation results relative to the halfway TS (target TS 54) obtained by performing the TS optimization calculation with respect to the halfway TS 53 are shown in FIG. 23, and its structure is shown in FIG. 24.
Data relative to the target TS 54 found by the TS-structure-optimization calculating unit 9 is readably stored in the storage device 5 by the TS-structure-optimization calculating unit 9 as target-TS data 31.
In FIG. 23, the first line shows keywords necessary for a calculation, the second line is a null line, the third line shows a title of the calculation, the fourth line is a null line, the fifth line shows an electric charge value and a spin value, and the sixth line and the following lines show atomic species, X coordinates, Y coordinates, and Z coordinates. Values, such as an energy value and so on, are shown in lines subsequent to the lines of atomic species and X, Y, and Z coordinates.

As described above, in the chemical reaction transition state search system 1 and the chemical reaction transition state search method according to the present embodiment, a search for a target TS can be made with a high probability while shortening a calculation time. Moreover, a target TS can be found even if it is a product having a complex molecular structure.
This is an effect achieved by undergoing a process in which a target TS (preliminary) is found by finding a halfway TS (preliminary) by use of a calculation (preliminary) model having low accuracy, thereafter a target TS is intended to be temporarily found from this target TS (preliminary), and, if the target TS is not found, a historically immediately previous halfway TS (preliminary) is read, thereafter a halfway TS is found from this stage by use of a calculation model having high accuracy this time, and a target TS is found from this halfway TS. In other words, this can be called the synergistic effect of the effect of the shortening of a calculation time obtained by continuously and economically performing a search method for two different target TSs and the effect of reliably finding a target TS having a complex molecular structure.
Additionally, to perform a calculation relative to TS optimization, a reactive site is first fixed, thereafter a structure whose all energy becomes minimum is optimized (steps S9 and S14), thereafter the fixed reactive site is unfixed, and a calculation relative to optimization of a TS whose all energy becomes maximum is performed (steps S10 and S15), and therefore the calculation operation can be performed efficiently and highly accurately.
Although the chemical reaction transition state search system and the chemical reaction transition state search method according to the present embodiment have been described as above, a description of the search system and the search method provided above can be used as a description of an embodiment of a program for a search for a transition state while allowing a general-purpose computer to perform the steps if FIG. 1 is regarded as showing the computer and if the flowchart of FIG. 2 is executed as a program according to which the computer operates. The operation and the effects according to the embodiment of this program are the same as the operation and the effects according to the embodiment of the chemical reaction transition state search system and that of the chemical reaction transition state search method described above.

### Industrial Applicability

As described above, the system, the method, and the program recited in claims 1 to 9 of the present invention are applicable to the pharmaceutical field, the chemical field, etc., in which new compounds are synthetically produced widely and generally for new drug development, pesticide development, etc.

## Claims

1. A chemical reaction transition state search system (1) including an input device (2), an arithmetic processing unit (3), and a storage device (5) in order to find a chemical structure being in a targeted transition state in a chemical reaction (hereinafter, a chemical structure being in a transition state is referred to as a transition-state geometry or a TS, and a transition-state geometry to be targeted is referred to as a target transition-state geometry or a target TS), **characterized in that** the arithmetic processing unit (3) includes:
an IG forming unit (7) that receives an input of an original structure of a halfway transition-state geometry being in a stage prior to the target TS (hereinafter, a "halfway transition-state geometry" is referred to specifically as a "halfway TS") and then calculates an initial geometry (hereinafter, referred to as an "IG") serving as a starting point to find the halfway TS from the halfway-TS original structure;
a CG-search calculating unit (8) that calculates a candidate geometry (hereinafter, referred to as a "CG") close to a transition state from an IG formed by the IG forming unit (7);
a TS optimization calculating unit (9) that determines whether an obtained TS is a true TS or is an original structure of a TS in such a manner that the TS is found by calculating a molecular structure that maximizes all energy while changing the molecular structure with respect to a CG calculated by the CG-search calculating unit (8) or with respect to the original structure of the TS calculated from the resulting CG, and the resulting TS is subjected to a vibration analysis by use of a vibration analytic function model (22);
a reactive-site fixing unit (10) that fixes a reactive site of the original structure of the TS;
a substituent processing unit (11) that adds or removes a substituent to or from the original structure of the TS; and
a structural optimization calculating unit (12) that calculates optimization of a molecular structure forming the original structure of the TS by calculating the molecular structure that minimizes all energy while changing the molecular structure;
and **characterized in that** the storage device (5) stores structure data (25, 26, 29, 30, 31) of the IG, of the CG, and of the TS, reactive-site data (28), and substituent data (27), and
the CG-search calculating unit (8), the TS optimization calculating unit (9), and the structural optimization calculating unit (12) selectively include both a functional model used for calculations and a functional model used to perform a calculation lower in accuracy than the functional model used for calculations.

2. A chemical reaction transition state search system (1) including an input device (2), an arithmetic processing unit (3), and a storage device (5) in order to find a chemical structure being in a targeted transition state in a chemical reaction (hereinafter, a chemical structure being in a transition state is referred to as a transition-state geometry or a TS, and a transition-state geometry to be targeted is referred to as a target transition-state geometry or a target TS), **characterized in that** the arithmetic processing unit (3) includes:
a halfway-TS setting unit (14) that calculates an original structure of a halfway transition-state geometry being in a stage prior to the target TS (hereinafter, a "halfway transition-state geometry" is referred to specifically as a "halfway TS") by receiving an input of data relative to a product of the chemical reaction;
an IG forming unit (7) that calculates an initial geometry (hereinafter, referred to as an "IG") serving as a starting point to find the halfway TS from the halfway-TS original structure set by the halfway-TS setting unit;
a CG-search calculating unit (8) that calculates a candidate geometry (hereinafter, referred to as a CG) close to a transition state from an IG formed by the IG forming unit (7);
a TS optimization calculating unit (9) that determines whether an obtained TS is a true TS or is an original structure of a TS in such a manner that the TS is found by calculating a molecular structure that maximizes all energy while changing the molecular structure with respect to a CG calculated by the CG-search calculating unit (8) or with respect to the original structure of the TS calculated from the resulting CG, and the resulting TS is subjected to a vibration analysis by use of a vibration analytic function model (22);
a reactive-site fixing unit (10) that fixes a reactive site of the original structure of the TS;
a substituent processing unit (11) that adds or removes a substituent to or from the original structure of the TS; and
a structural optimization calculating unit that calculates optimization of a molecular structure forming the original structure of the TS by calculating the molecular structure that minimizes all energy while changing the molecular structure;
and **characterized in that** the storage device (5) stores structure data (25, 26, 29, 30, 31) of the IG, of the CG, and of the TS, reactive-site data (28), and substituent data (27), and
the CG-search calculating unit (8), the TS optimization calculating unit (9), and the structural optimization calculating unit (12) selectively include both a functional model used for calculations and a functional model used to perform a calculation lower in accuracy than the functional model used for calculations.

3. The chemical reaction transition state search system (1) according to claim 1 or claim 2, **characterized by** further including an output device (4) that outputs or displays structure data relative to the TS that has been regarded as a true TS in the TS optimization calculating unit (9) to or on an external device.

4. A chemical reaction transition state search method for finding a chemical structure being in a targeted transition state in a chemical reaction (hereinafter, a chemical structure being in a transition state is referred to as a transition-state geometry or a TS, and a transition-state geometry to be targeted is referred to as a target transition-state geometry or a target TS), the chemical reaction transition state search method **characterized by**:
obtaining a halfway TS (hereinafter, a halfway TS obtained by a calculation (preliminary) is referred to as a "halfway TS (preliminary)" ) from an original structure of a halfway transition-state geometry being in a stage prior to the target TS (hereinafter, a "halfway transition-state geometry" is referred to specifically as a "halfway TS") by calculating a molecular structure that maximizes all energy while changing the molecular structure according to a calculation (preliminary) using a functional model lower in accuracy, and
obtaining the target TS by adding a substituent to the halfway TS (preliminary) so as to be a target TS (preliminary) and then by calculating the molecular structure that maximizes all energy while changing the molecular structure according to a calculation using a functional model higher in accuracy than the calculation (preliminary),
and **characterized by**:
if the target TS is not obtained, obtaining the halfway TS from the halfway TS (preliminary) by calculating the molecular structure that maximizes all energy while changing the molecular structure according to a calculation using the functional model higher in accuracy than the calculation (preliminary), and
obtaining the target TS by adding a substituent to the halfway TS and by calculating the molecular structure that maximizes all energy while changing the molecular structure according to a calculation using the functional model higher in accuracy than the calculation (preliminary).

5. A chemical reaction transition state search method for finding a chemical structure being in a targeted transition state in a chemical reaction (hereinafter, a chemical structure being in a transition state is referred to as a transition-state geometry or a TS, and a transition-state geometry to be targeted is referred to as a target transition-state geometry or a target TS), the chemical reaction transition state search method **characterized by**:
obtaining a halfway TS (hereinafter, a halfway TS obtained by a calculation (preliminary) is referred to as a "halfway TS (preliminary)") by calculating an original structure of a halfway transition-state geometry being in a stage prior to the target TS (hereinafter, a "halfway transition-state geometry" is referred to specifically as a "halfway TS") and by calculating a molecular structure that maximizes all energy while changing the molecular structure from the original structure of the halfway TS according to a calculation (preliminary) using a functional model lower in accuracy, and
obtaining the target TS by adding a substituent to the halfway TS (preliminary) so as to be a target TS (preliminary) and then by calculating the molecular structure that maximizes all energy while changing the molecular structure according to a calculation using a functional model higher in accuracy than the calculation (preliminary),
and **characterized by**:
if the target TS is not obtained, obtaining the halfway TS from the halfway TS (preliminary) by calculating the molecular structure that maximizes all energy while changing the molecular structure according to a calculation using the functional model higher in accuracy than the calculation (preliminary), and
obtaining the target TS by adding a substituent to the halfway TS and by calculating the molecular structure that maximizes all energy while changing the molecular structure according to a calculation using the functional model higher in accuracy than the calculation (preliminary).

6. A chemical reaction transition state search method for finding a chemical structure being in a targeted transition state in a chemical reaction (hereinafter, a chemical structure being in a transition state is referred to as a transition-state geometry or a TS, and a transition-state geometry to be targeted is referred to as a target transition-state geometry or a target TS), the chemical reaction transition state search method comprising:
an IG forming step (S1) of receiving an input of a halfway-TS original structure of a halfway transition-state geometry being in a stage prior to the target TS (hereinafter, a "halfway transition-state geometry" is referred to specifically as a "halfway TS") and then calculating an initial geometry (hereinafter, referred to as an "IG") serving as a starting point to find the halfway TS from the halfway-TS original structure;
a CG-search calculating step (S2) of calculating a candidate geometry (hereinafter, referred to as a "CG") close to a transition state from an IG formed by the IG forming step (S1) according to a calculation (preliminary) using a functional model lower in accuracy (hereinafter, a CG obtained by the calculation (preliminary) is referred to as a "CG (preliminary)");
a halfway TS (preliminary) optimization calculating step (S3) of obtaining a halfway TS (hereinafter, a halfway TS obtained according to the calculation (preliminary) is referred to as a "halfway TS (preliminary)") by calculating a molecular structure that maximizes all energy while changing the molecular structure with respect to the CG (preliminary) obtained by the CG-search calculating step (S2) according to the calculation (preliminary) using a functional model lower in accuracy;
a halfway TS (preliminary) determining step (S4) of determining whether an obtained halfway TS (preliminary) is a true halfway TS (preliminary) or is an original structure of a halfway TS (preliminary) in such a manner that the obtained halfway TS (preliminary) is subjected to a vibration analysis by use of a vibration analytic function model;
a writing step (S5) of readably writing a halfway TS (preliminary) obtained when it is determined that the obtained halfway TS (preliminary) is a halfway TS (preliminary) at the halfway TS (preliminary) determining step (S4) onto the storage device;
a target TS (preliminary) determining step (S6) of determining whether the halfway TS (preliminary) coincides with an original structure of a target TS (preliminary);
a target TS (preliminary) structural optimization calculating step (S9) of, when the halfway TS (preliminary) coincides with the original structure of the target TS (preliminary), calculating optimization of a molecular structure forming the target TS (preliminary) by fixing a reactive site of the target TS (preliminary) and by calculating the molecular structure that minimizes all energy while changing the molecular structure forming the target TS (preliminary) according to the calculation (preliminary) using a functional model lower in accuracy;
a target TS optimization calculating step (S10) of obtaining a target TS by unfixing the fixed reactive site of the target TS (preliminary) whose molecular structure has been optimized and by calculating a molecular structure that maximizes all energy while changing the molecular structure according to a calculation using a functional model higher in accuracy than the calculation (preliminary);
a first substituent adding step (S7) of adding a substituent to the halfway TS (preliminary) when the halfway TS (preliminary) does not coincide with the original structure of the target TS (preliminary) at the target TS (preliminary) determining step (S6);
a first substituent structural optimization calculating step (S8) of calculating optimization of a molecular structure of a substituent by calculating a molecular structure that minimizes all energy while changing the molecular structure with respect to only a site of the added substituent according to a calculation (preliminary) using a functional model lower in accuracy and then incorporating the resulting structure into the halfway TS (preliminary) optimization calculating step (S3) as a new halfway TS (preliminary);
a first target TS determining step (S11) of determining whether the target TS obtained at the target TS optimization calculating step (S10) is a true target TS or is an original structure of the target TS by performing a vibration analysis of the obtained target TS by use of a vibration analytic function model;
a halfway TS structural optimization calculating step (S14) of, when it is determined that the obtained target TS is the original structure of the target TS at the first target TS determining step (S11), calculating optimization of a molecular structure forming the halfway TS (preliminary) by performing a reading step (S13) of reading the halfway TS (preliminary) written onto the storage device at the writing step (S5), by fixing a reactive site of the halfway TS (preliminary), and by calculating the molecular structure that minimizes all energy while changing the molecular structure according to a calculation using a functional model higher in accuracy than the calculation (preliminary);
a first halfway TS optimization calculating step (S15) of obtaining a halfway TS by unfixing the fixed reactive site of the halfway TS whose molecular structure has been optimized and by calculating the molecular structure that maximizes all energy while changing the molecular structure according to a calculation using a functional model higher in accuracy than the calculation (preliminary);
a first halfway TS determining step (S16) of determining whether an obtained halfway TS is a true halfway TS or is an original structure of the halfway TS by performing a vibration analysis of the obtained halfway TS by use of a vibration analytic function model and, when it is determined that the obtained halfway TS is the original structure of the halfway TS, incorporating a halfway TS (preliminary) written prior to the halfway TS (preliminary) written onto the storage device into the reading step (S13);
a second target TS determining step (S18) of, when it is determined (S17) that the obtained halfway TS is a true halfway TS at the first halfway TS determining step (S16), determining whether the halfway TS coincides with the target TS;
a second substituent adding step (S19) of, when it is determined that the halfway TS does not coincide with the target TS at the second target TS determining step (S18), adding a substituent to the halfway TS;
a second substituent structural optimization calculating step (S20) of calculating optimization of a molecular structure of a substituent by calculating a molecular structure that minimizes all energy while changing the molecular structure with respect to only a site of the added substituent according to a calculation using a functional model higher in accuracy than the calculation (preliminary);
a second halfway TS optimization calculating step (S21) of obtaining a halfway TS by calculating a molecular structure that maximizes all energy while changing the molecular structure according to a calculation using a functional model higher in accuracy than the calculation (preliminary) while setting the halfway TS optimized at the second substituent structural optimization calculating step as a new halfway TS; and
a second halfway TS determining step (S22) of determining whether the halfway TS obtained at the second halfway TS optimization calculating step (S21) is a true halfway TS or is an original structure of the halfway TS by performing a vibration analysis of the obtained halfway TS by use of a vibration analytic function model and, when it is determined that the obtained halfway TS is a true halfway TS, incorporating the halfway TS into the second target TS determining step (S18).

7. A chemical reaction transition state search method for finding a chemical structure being in a targeted transition state in a chemical reaction (hereinafter, a chemical structure being in a transition state is referred to as a transition-state geometry or a TS, and a transition-state geometry to be targeted is referred to as a target transition-state geometry or a target TS), the chemical reaction transition state search method comprising:
a halfway TS setting step (S0) of calculating an original structure of a halfway transition-state geometry being in a stage prior to the target TS (hereinafter, a "halfway transition-state geometry" is referred to specifically as a "halfway TS");
an IG forming step (S1) of calculating an initial geometry (hereinafter, referred to as an "IG") that serves as a starting point to find the halfway TS from the halfway-TS original structure set at the halfway TS setting step (S0);
a CG-search calculating step (S2) of calculating a candidate geometry (hereinafter, referred to as a "CG") close to a transition state from an IG formed by the IG forming step (S1) according to a calculation (preliminary) using a functional model lower in accuracy (hereinafter, a CG obtained by the calculation (preliminary) is referred to as a "CG (preliminary)");
a halfway TS (preliminary) optimization calculating step (S3) of obtaining a halfway TS (hereinafter, a halfway TS obtained according to the calculation (preliminary) is referred to as a "halfway TS (preliminary)") by calculating a molecular structure that maximizes all energy while changing the molecular structure with respect to the CG (preliminary) obtained by the CG-search calculating step (S2) according to the calculation (preliminary) using a functional model lower in accuracy;
a halfway TS (preliminary) determining step (S4) of determining whether an obtained halfway TS (preliminary) is a true halfway TS (preliminary) or is an original structure of a halfway TS (preliminary) in such a manner that the obtained halfway TS (preliminary) is subjected to a vibration analysis by use of a vibration analytic function model;
a writing step (S5) of readably writing a halfway TS (preliminary) obtained when it is determined that the obtained halfway TS (preliminary) is a halfway TS (preliminary) at the halfway TS (preliminary) determining step (S4) onto the storage device;
a target TS (preliminary) determining step (S6) of determining whether the halfway TS (preliminary) coincides with an original structure of a target TS (preliminary);
a target TS (preliminary) structural optimization calculating step (S9) of, when the halfway TS (preliminary) coincides with the original structure of the target TS (preliminary), calculating optimization of a molecular structure forming the target TS (preliminary) by fixing a reactive site of the target TS (preliminary) and by calculating the molecular structure that minimizes all energy while changing the molecular structure forming the target TS (preliminary) according to the calculation (preliminary) using a functional model lower in accuracy;
a target TS optimization calculating step (S10) of obtaining a target TS by unfixing the fixed reactive site of the target TS (preliminary) whose molecular structure has been optimized and by calculating a molecular structure that maximizes all energy while changing the molecular structure according to a calculation using a functional model higher in accuracy than the calculation (preliminary);
a first substituent adding step (S7) of adding a substituent to the halfway TS (preliminary) when the halfway TS (preliminary) does not coincide with the original structure of the target TS (preliminary) at the target TS (preliminary) determining step (S6);
a first substituent structural optimization calculating step (S8) of calculating optimization of a molecular structure of a substituent by calculating a molecular structure that minimizes all energy while changing the molecular structure with respect to only a site of the added substituent according to a calculation (preliminary) using a functional model lower in accuracy and then incorporating the resulting structure into the halfway TS (preliminary) optimization calculating step (S3) as a new halfway TS (preliminary);
a first target TS determining step (S11) of determining whether the target TS obtained at the target TS optimization calculating step (S10) is a true target TS or is an original structure of the target TS by performing a vibration analysis of the obtained target TS by use of a vibration analytic function model;
a halfway TS structural optimization calculating step (S14) of, when it is determined that the obtained target TS is the original structure of the target TS at the first target TS determining step (S11), calculating optimization of a molecular structure forming the halfway TS (preliminary) by performing a reading step (S13) of reading the halfway TS (preliminary) written onto the storage device at the writing step (S5), by fixing a reactive site of the halfway TS (preliminary), and by calculating the molecular structure that minimizes all energy while changing the molecular structure according to a calculation using a functional model higher in accuracy than the calculation (preliminary);
a first halfway TS optimization calculating step (S15) of obtaining a halfway TS by unfixing the fixed reactive site of the halfway TS whose molecular structure has been optimized and by calculating the molecular structure that maximizes all energy while changing the molecular structure according to a calculation using a functional model higher in accuracy than the calculation (preliminary);
a first halfway TS determining step (S16) of determining whether an obtained halfway TS is a true halfway TS or is an original structure of the halfway TS by performing a vibration analysis of the obtained halfway TS by use of a vibration analytic function model and, when it is determined that the obtained halfway TS is the original structure of the halfway TS, incorporating a halfway TS (preliminary) written prior to the halfway TS (preliminary) written onto the storage device into the reading step (S13);
a second target TS determining step (S18) of, when it is determined (S17) that the obtained halfway TS is a true halfway TS at the first halfway TS determining step (S16), determining whether the halfway TS coincides with the target TS;
a second substituent adding step (S19) of, when it is determined that the halfway TS does not coincide with the target TS at the second target TS determining step (S18), adding a substituent to the halfway TS;
a second substituent structural optimization calculating step (S20) of calculating optimization of a molecular structure of a substituent by calculating a molecular structure that minimizes all energy while changing the molecular structure with respect to only a site of the added substituent according to a calculation using a functional model higher in accuracy than the calculation (preliminary);
a second halfway TS optimization calculating step (S21) of obtaining a halfway TS by calculating a molecular structure that maximizes all energy while changing the molecular structure according to a calculation using a functional model higher in accuracy than the calculation (preliminary) while setting the halfway TS optimized at the second substituent structural optimization calculating step as a new halfway TS; and
a second halfway TS determining step (S22) of determining whether the halfway TS obtained at the second halfway TS optimization calculating step (S21) is a true halfway TS or is an original structure of the halfway TS by performing a vibration analysis of the obtained halfway TS by use of a vibration analytic function model and, when it is determined that the obtained halfway TS is a true halfway TS, incorporating the halfway TS into the second target TS determining step (S18).

8. A chemical reaction transition state search program executed by a computer in order to find a chemical structure being in a targeted transition state in a chemical reaction (hereinafter, a chemical structure being in a transition state is referred to as a transition-state geometry or a TS, and a transition-state geometry to be targeted is referred to as a target transition-state geometry or a target TS), the chemical reaction transition state search program allowing the computer to perform:
an IG forming step (S1) of receiving an input of an original structure of a halfway transition-state geometry being in a stage prior to the target TS (hereinafter, a "halfway transition-state geometry" is referred to specifically as a "halfway TS") and then calculating an initial geometry (hereinafter, referred to as an "IG") that serves as a starting point to find the halfway TS from the halfway-TS original structure;
a CG-search calculating step (S2) of calculating a candidate geometry (hereinafter, referred to as a "CG") close to a transition state from an IG formed by the IG forming step (S1) according to a calculation (preliminary) using a functional model lower in accuracy (hereinafter, a CG obtained by the calculation (preliminary) is referred to as a "CG (preliminary)");
a halfway TS (preliminary) optimization calculating step (S3) of obtaining a halfway TS (hereinafter, a halfway TS obtained according to the calculation (preliminary) is referred to as a "halfway TS (preliminary)") by calculating a molecular structure that maximizes all energy while changing the molecular structure with respect to the CG (preliminary) obtained by the CG-search calculating step (S2) according to the calculation (preliminary) using a functional model lower in accuracy;
a halfway TS (preliminary) determining step (S4) of determining whether an obtained halfway TS (preliminary) is a true halfway TS (preliminary) or is an original structure of a halfway TS (preliminary) in such a manner that the obtained halfway TS (preliminary) is subjected to a vibration analysis by use of a vibration analytic function model;
a writing step (S5) of readably writing a halfway TS (preliminary) obtained when it is determined that the obtained halfway TS (preliminary) is a halfway TS (preliminary) at the halfway TS (preliminary) determining step (S4) onto the storage device;
a target TS (preliminary) determining step (S6) of determining whether the halfway TS (preliminary) coincides with an original structure of a target TS (preliminary);
a target TS (preliminary) structural optimization calculating step (S9) of, when the halfway TS (preliminary) coincides with the original structure of the target TS (preliminary), calculating optimization of a molecular structure forming the target TS (preliminary) by fixing a reactive site of the target TS (preliminary) and by calculating the molecular structure that minimizes all energy while changing the molecular structure forming the target TS (preliminary) according to the calculation (preliminary) using a functional model lower in accuracy;
a target TS optimization calculating step (S10) of obtaining a target TS by unfixing the fixed reactive site of the target TS (preliminary) whose molecular structure has been optimized and by calculating a molecular structure that maximizes all energy while changing the molecular structure according to a calculation using a functional model higher in accuracy than the calculation (preliminary);
a first substituent adding step (S7) of adding a substituent to the halfway TS (preliminary) when the halfway TS (preliminary) does not coincide with the original structure of the target TS (preliminary) at the target TS (preliminary) determining step (S6);
a first substituent structural optimization calculating step (S8) of calculating optimization of a molecular structure of a substituent by calculating a molecular structure that minimizes all energy while changing the molecular structure with respect to only a site of the added substituent according to a calculation (preliminary) using a functional model lower in accuracy and then incorporating the resulting structure into the halfway TS (preliminary) optimization calculating step (S3) as a new halfway TS (preliminary);
a first target TS determining step (S11) of determining whether the target TS obtained at the target TS optimization calculating step (S10) is a true target TS or is an original structure of the target TS by performing a vibration analysis of the obtained target TS by use of a vibration analytic function model;
a halfway TS structural optimization calculating step (S14) of, when it is determined that the obtained target TS is the original structure of the target TS at the first target TS determining step (S11), calculating optimization of a molecular structure forming the halfway TS (preliminary) by performing a reading step (S13) of reading the halfway TS (preliminary) written onto the storage device at the writing step (S5), by fixing a reactive site of the halfway TS (preliminary), and by calculating the molecular structure that minimizes all energy while changing the molecular structure according to a calculation using a functional model higher in accuracy than the calculation (preliminary);
a first halfway TS optimization calculating step (S15) of obtaining a halfway TS by unfixing the fixed reactive site of the halfway TS whose molecular structure has been optimized and by calculating the molecular structure that maximizes all energy while changing the molecular structure according to a calculation using a functional model higher in accuracy than the calculation (preliminary);
a first halfway TS determining step (S16) of determining whether an obtained halfway TS is a true halfway TS or is an original structure of the halfway TS by performing a vibration analysis of the obtained halfway TS by use of a vibration analytic function model and, when it is determined that the obtained halfway TS is the original structure of the halfway TS, incorporating a halfway TS (preliminary) written prior to the halfway TS (preliminary) written onto the storage device into the reading step (S13);
a second target TS determining step (S18) of, when it is determined (S17) that the obtained halfway TS is a true halfway TS at the first halfway TS determining step (S16), determining whether the halfway TS coincides with the target TS;
a second substituent adding step (S19) of, when it is determined that the halfway TS does not coincide with the target TS at the second target TS determining step (S18), adding a substituent to the halfway TS;
a second substituent structural optimization calculating step (S20) of calculating optimization of a molecular structure of a substituent by calculating a molecular structure that minimizes all energy while changing the molecular structure with respect to only a site of the added substituent according to a calculation using a functional model higher in accuracy than the calculation (preliminary);
a second halfway TS optimization calculating step (S21) of obtaining a halfway TS by calculating a molecular structure that maximizes all energy while changing the molecular structure according to a calculation using a functional model higher in accuracy than the calculation (preliminary) while setting the halfway TS optimized at the second substituent structural optimization calculating step as a new halfway TS; and
a second halfway TS determining step (S22) of determining whether the halfway TS obtained at the second halfway TS optimization calculating step (S21) is a true halfway TS or is an original structure of the halfway TS by performing a vibration analysis of the obtained halfway TS by use of a vibration analytic function model and, when it is determined that the obtained halfway TS is a true halfway TS, incorporating the halfway TS into the second target TS determining step (S18).

9. A chemical reaction transition state search program executed by a computer in order to find a chemical structure being in a targeted transition state in a chemical reaction (hereinafter, a chemical structure being in a transition state is referred to as a transition-state geometry or a TS, and a transition-state geometry to be targeted is referred to as a target transition-state geometry or a target TS), the chemical reaction transition state search program allowing the computer to perform:
a halfway TS setting step (S0) of calculating an original structure of a halfway transition-state geometry being in a stage prior to the target TS (hereinafter, a "halfway transition-state geometry" is referred to specifically as a "halfway TS");
an IG forming step (S1) of calculating an initial geometry (hereinafter, referred to as an "IG") that serves as a starting point to find the halfway TS from the halfway-TS original structure set at the halfway TS setting step (S0);
a CG-search calculating step (S2) of calculating a candidate geometry (hereinafter, referred to as a "CG") close to a transition state from an IG formed by the IG forming step (S1) according to a calculation (preliminary) using a functional model lower in accuracy (hereinafter, a CG obtained by the calculation (preliminary) is referred to as a "CG (preliminary)");
a halfway TS (preliminary) optimization calculating step (S3) of obtaining a halfway TS (hereinafter, a halfway TS obtained according to the calculation (preliminary) is referred to as a "halfway TS (preliminary)") by calculating a molecular structure that maximizes all energy while changing the molecular structure with respect to the CG (preliminary) obtained by the CG-search calculating step (S2) according to the calculation (preliminary) using a functional model lower in accuracy;
a halfway TS (preliminary) determining step (S4) of determining whether an obtained halfway TS (preliminary) is a true halfway TS (preliminary) or is an original structure of a halfway TS (preliminary) in such a manner that the obtained halfway TS (preliminary) is subjected to a vibration analysis by use of a vibration analytic function model;
a writing step (S5) of readably writing a halfway TS (preliminary) obtained when it is determined that the obtained halfway TS (preliminary) is a halfway TS (preliminary) at the halfway TS (preliminary) determining step (S4) onto the storage device;
a target TS (preliminary) determining step (S6) of determining whether the halfway TS (preliminary) coincides with an original structure of a target TS (preliminary);
a target TS (preliminary) structural optimization calculating step (S9) of, when the halfway TS (preliminary) coincides with the original structure of the target TS (preliminary), calculating optimization of a molecular structure forming the target TS (preliminary) by fixing a reactive site of the target TS (preliminary) and by calculating the molecular structure that minimizes all energy while changing the molecular structure forming the target TS (preliminary) according to the calculation (preliminary) using a functional model lower in accuracy;
a target TS optimization calculating step (S10) of obtaining a target TS by unfixing the fixed reactive site of the target TS (preliminary) whose molecular structure has been optimized and by calculating a molecular structure that maximizes all energy while changing the molecular structure according to a calculation using a functional model higher in accuracy than the calculation (preliminary);
a first substituent adding step (S7) of adding a substituent to the halfway TS (preliminary) when the halfway TS (preliminary) does not coincide with the original structure of the target TS (preliminary) at the target TS (preliminary) determining step (S6);
a first substituent structural optimization calculating step (S8) of calculating optimization of a molecular structure of a substituent by calculating a molecular structure that minimizes all energy while changing the molecular structure with respect to only a site of the added substituent according to a calculation (preliminary) using a functional model lower in accuracy and then incorporating the resulting structure into the halfway TS (preliminary) optimization calculating step (S3) as a new halfway TS (preliminary);
a first target TS determining step (S11) of determining whether the target TS obtained at the target TS optimization calculating step (S10) is a true target TS or is an original structure of the target TS by performing a vibration analysis of the obtained target TS by use of a vibration analytic function model;
a halfway TS structural optimization calculating step (S14) of, when it is determined that the obtained target TS is the original structure of the target TS at the first target TS determining step (S11), calculating optimization of a molecular structure forming the halfway TS (preliminary) by performing a reading step (S13) of reading the halfway TS (preliminary) written onto the storage device at the writing step (S5), by fixing a reactive site of the halfway TS (preliminary), and by calculating the molecular structure that minimizes all energy while changing the molecular structure according to a calculation using a functional model higher in accuracy than the calculation (preliminary);
a first halfway TS optimization calculating step (S15) of obtaining a halfway TS by unfixing the fixed reactive site of the halfway TS whose molecular structure has been optimized and by calculating the molecular structure that maximizes all energy while changing the molecular structure according to a calculation using a functional model higher in accuracy than the calculation (preliminary);
a first halfway TS determining step (S16) of determining whether an obtained halfway TS is a true halfway TS or is an original structure of the halfway TS by performing a vibration analysis of the obtained halfway TS by use of a vibration analytic function model and, when it is determined that the obtained halfway TS is the original structure of the halfway TS, incorporating a halfway TS (preliminary) written prior to the halfway TS (preliminary) written onto the storage device into the reading step (S13);
a second target TS determining step (S18) of, when it is determined (S17) that the obtained halfway TS is a true halfway TS at the first halfway TS determining step (S16), determining whether the halfway TS coincides with the target TS;
a second substituent adding step (S19) of, when it is determined that the halfway TS does not coincide with the target TS at the second target TS determining step (S18), adding a substituent to the halfway TS;
a second substituent structural optimization calculating step (S20) of calculating optimization of a molecular structure of a substituent by calculating a molecular structure that minimizes all energy while changing the molecular structure with respect to only a site of the added substituent according to a calculation using a functional model higher in accuracy than the calculation (preliminary);
a second halfway TS optimization calculating step (S21) of obtaining a halfway TS by calculating a molecular structure that maximizes all energy while changing the molecular structure according to a calculation using a functional model higher in accuracy than the calculation (preliminary) while setting the halfway TS optimized at the second substituent structural optimization calculating step as a new halfway TS; and
a second halfway TS determining step (S22) of determining whether the halfway TS obtained at the second halfway TS optimization calculating step (S21) is a true halfway TS or is an original structure of the halfway TS by performing a vibration analysis of the obtained halfway TS by use of a vibration analytic function model and, when it is determined that the obtained halfway TS is a true halfway TS, incorporating the halfway TS into the second target TS determining step (S18).
